# EUROPEAN PATENT APPLICATION

(11) **EP 4 556 068 A2**
(43) Date of publication of application: **21.05.2025**
(21) Application number: 25169181.2
(22) Date of filing: 18.04.2017
(51) Int. Cl.: A61P 25/08

(54) **ORALLY INHALED AND NASAL BENZODIAZEPINES**

(30) Priority: 14.04.2016 LU 93027
(62) Divisional of application: 17721067.1
(71) Applicant: Paion UK Limited, Cambridge, Cambridgeshire CB4 2QH (GB)
(72) Inventor: Petersen, Karl-Uwe, 52072 Aachen (DE); Sakata, Derek Jo, Salt Lake City, 84117 (US); Stöhr, Thomas, 2400 Mol (BE); Graham, John, Over Cambridgeshire, CB4 5NE (GB); Cooper, Brett, Cheshunt Hertfordshire, EN8 9HH (GB); Bevans, Tatjana, Salt Lake City, Utah, 84112 (US); Reilly, Christopher, Salt Lake City, Utah, 84112 (US)
(74) Representative: Hoffmann Eitle

(57) **Abstract**

The present invention relates to Orally Inhaled and Nasal Drug Product (OINDP) comprising a benzodiazepine, in particular remimazolam.

## Description

The present invention relates to certain benzodiazepines or pharmaceutically acceptable salts thereof for use as an Orally Inhaled and Nasal Drug Product (OINDP).

Benzodiazepine compounds are known for their capacity to bind to a site on a specific receptor/chloride ion channel complex known as the GABA_{A} receptor. The binding of a benzodiazepine potentiates the binding of the inhibitory neurotransmitter gamma-aminobutyric acid (GABA) to the complex, thereby leading to inhibition of normal neuronal function. Therapeutic purposes of the treatment with benzodiazepine compounds are in particular production of sedation or hypnosis, induction of anxiolysis, induction of muscle relaxation, treatment of convulsions or induction and/or maintenance of anesthesia in a mammal. See generally, Goodman and Gilman's The Pharmacological Basis of Therapeutics, Eighth Edition; Gilman, A. G.; Rall, T. W.; Nies, A. S.; Taylor, P., Eds. ; Pergamon Press: New York, 1990; pp. 303-304, 346-358.

Short-acting benzodiazepines that may provide faster recovery profiles have been the subject of clinical investigations (W. Hering et al., Anesthesiology 1996, 189, 85 (Suppl.); J. Dingemanse et al., Br. J. Anaesth 1997, 79, 567-574). Further compounds of interest are disclosed in WO 96/23790, WO 96/20941 and US 5665 718. Other publications that describe benzodiazepinones include E. Manghisi and A. Salimbemi, Boll. Chim. Farm. 1974, 113, 642-644, W. A. Khan and P. Singh, Org. Prep. Proc. Int. 1978, 10,105-111 and J. B. Hester, Jr, et al., J. Med. Chem. 1980, 23, 643-647. Benzodiazepines such as diazepam, lorazepam, and midazolam all undergo metabolism by hepatic-dependent processes. Active metabolites, which are often much more slowly metabolized than the parent drug, can be generated by these hepatic mechanisms in effect prolonging the duration of action of many benzodiazepines (T. M. Bauer et al, Lancet 1995, 346, 145-7). Inadvertent oversedation has been associated with the use of benzodiazepines (A. Shafer, Crit Care Med 1998, 26, 947-956), particularly in the intensive care unit, where benzodiazepines, such as midazolam, enjoy frequent use.

Benzodiazepines are conventionally administered intravenously (IV) or orally. The IV route has however some disadvantages. For example, medical personnel are usually required for administering the drug which poses burden on the health care system. Self-administration by patients may result in low patient compliance. Strict hygienic conditions are required for preparing injection and special care should be taken to dispose needles. In particular younger patients fear pain associated with injections. Therefore, routes would be desirable which overcome at least one of the disadvantages of IV administration.

Recently, it was suggested to administer certain benzodiazepines intranasally or intrapulmonary. For example, midazolam (see formula below) is said to be efficacious when administered intranasally (Wermeling et al. Epilepsy Research. 2009. 83:124-132). Intrapulmonary midazolam is described in US patent application 2013/0309306 A1. Until now, there is however no commercial midazolam product utilizing the intranasal and intrapulmonary route. Low tolerability of intranasally given midazolam as reported in the literature (see, e.g., Veldhorst-Janssen et al. Clinical Therapeutics. 2011. 33(12):2022-2028; Ivaturi et al. Epilepsy Research. 2009. 84:120-126) might be one explanation for this.

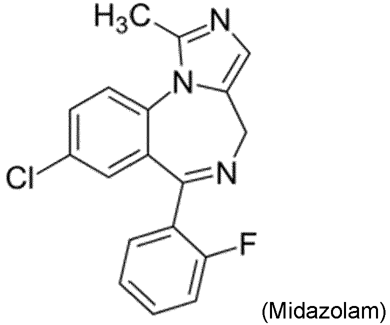

Besides a lack of tolerability there are further limitations associated with the intranasal and intrapulmonary routes of administration. For example, intranasal application often suffers from low and highly variable bioavailability, removal of the drug by mucociliary clearance, nonspecific defense of respiratory organs and enzymatic degradation. Active ingredients may be metabolized in the nasal cavity during their passage through the epithelium due to the presence of a wide spectrum of enzymes, including tissue esterases. These limitations are a particular concern when a fast onset of the drug - such as for example with benzodiazepines - is desired.

Therefore, it is the object of the present invention to provide a medicament comprising a benzodiazepine which overcomes at least one of the problems of IV administration. Preferably, it is well tolerated and efficacious. This type of administration should preferably lead to a better patient compliance.

In contrast to intranasal midazolam the inventors found that intranasal and intrapulmonary delivery of the known benzodiazepine remimazolam is well tolerated. Moreover, the inventors found that remimazolam administered by this route is efficacious. Given remimazolam (see formula below) belongs to a group of benzodiazepines which comprise a carboxylic acid ester moiety, this finding was surprising. When such benzodiazepines are to penetrate the mucosal tissue the prior art (e.g., WO 2013/174883 A1) suggested them to be inactivated by tissue esterases. Prior to the inventor's finding remimazolam was thus not expected to be efficacious when administered by the intranasal and intrapulmonary route.

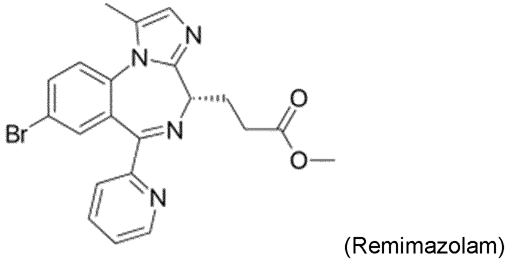

These findings advantageously allow for a simple and convenient administration. The application may be performed as a painless method, which is thus particular suitable for infants (up to 12 months of age), children (1 to 12 years of age), and adolescents (12 to 17 years of age), in particular infants and children. It does not require sterile conditions, and may be easily controlled by the patient or other medicinally unskilled personnel.

The present invention relates to benzodiazepines according to formula (I) 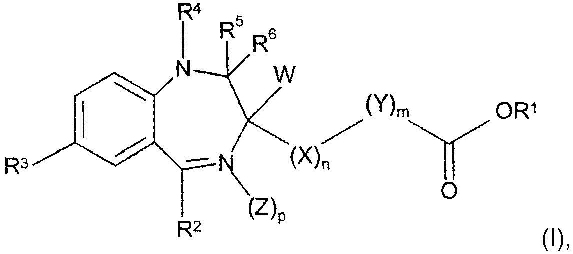 wherein
- W is H;
- X is CH₂; n is 1;
- Y is CH₂; m is 1;
- Z is 0; p is 0 or 1;
- R¹ is CH₃, CH₂CH₃, CH₂CH₂CH₃, CH(CH₃)₂ or CH₂CH(CH₃)₂;
- R² is 2-fluorophenyl, 2-chlorophenyl or 2-pyridyl;
- R³ is CI or Br;
- R⁴, R⁵ and R⁶ form the group-CR⁸=U-V= wherein R³ is hydrogen, C₁₋₄ alkyl or C₁₋₃ hydroxyalkyl, U is N or CR⁹ wherein R⁹ is H, C₁₋₄ alkyl, C₁₋₃ hydroxyalkyl or C₁₋₄ alkoxy, V is N or CH and p is zero,
or a pharmaceutically acceptable salt thereof.

The most preferred embodiment is remimazolam (and salts thereof, preferably remimazolam besylate, remimazolam esylate, or remimazolam tosylate, in particular remimazolam besylate). Unless otherwise explicitly mentioned hereinafter the term remimazolam always includes salts thereof.

According to the invention benzodiazepines of the invention are used as a medicament, specifically an orally inhaled and nasal drug product (OINDP). OINDPs are defined by the International Pharmaceutical Aerosol Consortium on Regulation & Science (IPAC-RS) as providing therapeutic benefit by delivery of a pharmaceutical substance to the lungs or nasal cavity. Both of these routes of administration by OINDP have common characteristics, in particular such as:
- delivery of the drug at a specific range of particle sizes, which may be the drug particle alone, or bound to a carrier (in particular a particulate carrier), or dissolved or suspended in a liquid droplet.
- targeted deposition to specific membranes (for example specific point of pulmonary tract, specific mucous membrane in the nasal cavity).

Accordingly, an OINDP is defined herein as medicament which is intended for administration of a drug substance to the respiratory tract, in particular to the lung and/or nasal structures. Preferably the definition of the OINDP further relates to a dosage form (for example a nasal spray, a nasal gel, a nasal ointment, inhalation solutions, inhalation suspensions, inhalation sprays, dry powder or an aerosol) which is specifically designed or adapted for administration of a drug substance to the respiratory tract, in particular to the lung and/or nasal structures. Hence in one aspect the invention relates to the benzodiazepine of the invention for use in a therapeutic method, wherein the drug substance is administered intranasally or intrapulmonary. Preferably the amount of benzodiazepine absorbed by structures other than the structures of the respiratory tract does not essentially contribute to the therapeutic effect of the administered benzodiazepine.

Administration to the respiratory tract means that the drug substance is substantially absorbed by structures of the respiratory tract in order to reach a therapeutic effect. Hence, upon administration a pharmaceutically effective amount of the drug substance is absorbed by the respiratory tract without undergoing substantial metabolic inactivation. The respiratory tract herein denotes the air passage from the nose to the pulmonary system (including the larynx, trachea, bronchi and/or alveoli, but preferably excluding the pharynx).

In the most preferred embodiment, the OINDP (or the drug substance) is administered by the intranasal or intrapulmonary route. Intranasal administration as used herein is defined as administration via the nasal structures, preferably through the nasal cavity, thereby enabling the absorption of a therapeutically effective amount of drug substance through the nasal structures. Pharmacologically active amounts of the OINDP or the drug substance are thereby delivered to the circulation or directly to the site of action i.e. the central nervous system via nasal to brain uptake. Preferably the amount of benzodiazepine absorbed by structures other than the nasal structures does not essentially contribute to the therapeutic effect of the administered benzodiazepine.

Intrapulmonary administration herein describes administration by entering the lungs and preferably means absorption to the lungs. Intrapulmonary administration means that a therapeutically effective amount of the drug substance is absorbed through structures of the pulmonary system. Preferably the amount of benzodiazepine absorbed by structures other than the structures of the pulmonary system does not essentially contribute to the therapeutic effect of the administered benzodiazepine.

Delivery of the benzodiazepines to the lung or nasal structures can be accomplished e.g. by inhaling, nebulization, snorting or applying the benzodiazepines directly into the nasal cavity e.g. as a cream.

According to the invention the benzodiazepines can therefore be used in a therapeutic method comprising the step of administering to a patient in need thereof a therapeutically effective amount of at least one benzodiazepine of the invention to the respiratory tract, preferably by intranasal or intrapulmonary administration. The present invention also relates to a benzodiazepine of the invention, in particular remimazolam or a salt thereof, for use by administration to the respiratory tract, preferably by intrapulmonary and/or intranasal administration of the benzodiazepine or salt.

It will be understood that the term "the benzodiazepine(s)" as used herein refers to the benzodiazepine(s) of formula I or its/their pharmaceutically acceptable salt(s) as defined herein unless otherwise explicitly mentioned.

The definition of "pharmaceutically acceptable" is meant to encompass any substance which does not unacceptably (preferably not at all) interfere with effectiveness of the biological activity of the active ingredient and that is not unacceptably (preferably not at all) toxic to the host to which it is administered.

In a further aspect, the present invention relates to a device including a benzodiazepine of the invention, in particular remimazolam. According to the invention, the device is adapted to administer an orally inhaled or nasal drug product. The device therefore includes the drug product (e.g. a gel or a dry powder) or produces the drug product (e.g. a spray or an aerosol) so that it can be administered by the intranasal or intrapulmonary route of administration.

A further aspect of the invention is a composition comprising the benzodiazepine of the invention, in particular remimazolam, and at least one of substances (a) to (c): (a) a propellant, e.g. a chlorofluorocarbon, hydrocarbon, hydrochlorofluorocarbon, hydrofluorocarbon or a compressed gas; (b) nano- or microparticles as defined herein and/or (c) a mucoadhesive as defined herein. A composition comprising polyethylene glycol (PEG), particularly PEG 400 is preferred.

Such compositions are not only suitable for the intranasal or intrapulmonary route of administration. They are also suitable for the reconstitution of solid drug substance, since they allow a fast reconstitution and result in a pharmaceutical composition wherein the drug substance remains dissolved. This in particular applies to an aqueous composition comprising PEG 400, preferably when used in 10 to 20 % (w/w). See example 3 *infra.* Such compositions can also be used for the preparation of a pharmaceutical composition for the intravenous administration of the benzodiazepines of the invention, in particular for remimazolam (including salts thereof).

In a further aspect, the present invention relates to an Orally Inhaled and Nasal Drug Product (OINDP) containing the benzodiazepine of the invention, in particular remimazolam.

Use of the benzodiazepine as defined herein, or a pharmaceutically acceptable salt thereof, in particular remimazolam, for the manufacture of an OINDP as defined herein is another aspect of the invention.

A further aspect relates to a method for inducing or maintaining sedation, hypnosis, anxiolysis, anesthesia, muscle relaxation or treating convulsions in a patient, comprising administering to the patient an effective amount of a benzodiazepine of the invention by intranasal or intrapulmonary route of administration, thereby inducing or maintaining sedation, hypnosis, anxiolysis, anesthesia, muscle relaxation or treating convulsions.

The embodiments described in the following shall be understood to describe preferred embodiments of benzodiazepines' use as a medicament (OINDP), their use by administration to the respiratory tract and in particular their use by intranasal and/or intrapulmonary administration. Further, the embodiments described, in particular the benzodiazepine and the formulations shall be understood as further defining the benzodiazepines contained in the compositions of the invention, in the devices of the invention or the OINDPs of the invention. Therefore the present invention includes compositions, devices and OINDPs which contain the benzodiazepines described in the various embodiments herein as well as compositions, devices and OINDPs which contain the formulations according to the herein described embodiments..

### Intranasal and intrapulmonary administration

To exert its therapeutic effect the benzodiazepine of the invention should enter the central nervous system (CNS). It may be transported via the blood circulation to the CNS. When the compound is administered via the intranasal or intrapulmonary route, it is applied to the nasal or intrapulmonary mucosa where it is absorbed and then transferred to the systemic blood circulation. This has the advantage of avoidance of a first-pass hepatic and intestinal metabolism.

Thus, in one embodiment the benzodiazepines are administered to a patient, for example a patient in need of treatment with a benzodiazepine of the invention, in particular remimazolam, to obtain a systemic effect in the patient. A systemic effect distinguishes from a non-systemic or local effect and describes a pharmacological effect that does not only affect parts of the body (e.g. the part where the drug is applied in topical administration). The systemic effect transfers via the distribution of the drug substance in the blood circulation essentially throughout the whole body.

In order to obtain a systemic effect at least 10 %, preferably at least 20 %, more preferably at least 30 %, most preferably at least 40 %, in particular at least 50 %, 60 %, 70 %, 80 % or even 90 % of the administered dose of the benzodiazepines should enter the blood circulation.

Alternatively, the benzodiazepines can be delivered directly from the nose to the brain. Nose-to-brain transferral is thought to be effected by the drug substance travelling along the olfactory nerve cells. The olfactory epithelium is situated in the upper posterior part of the human nasal cavity. The nerve cells of the olfactory epithelium project into the olfactory bulb of the brain, which provides a direct connection between the brain and the external environment.

Thus, in another embodiment the benzodiazepines are administered to the patient in order to obtain a non-systemic effect in the patient. A non-systemic effect describes a medical treatment that affects only part of the body, and preferably affects essentially only the brain. To obtain a non-systemic effect less than 90 %, preferably less than 80 %, more preferably less than 70 %, most preferably less than 60 %, in particular less than 50 %, 40 %, 30 %, 20 % or even 10 % of the administered dose of the benzodiazepines should enter the blood circulation. The benzodiazepines may travel from the brain to the systemic circulation where they are eliminated by the liver and/or kidney. These benzodiazepines will usually not cause a therapeutic effect. Thus, the above percentages should be understood as defining the fraction of benzodiazepines that enter the blood circulation before entering the brain, e.g. those benzodiazepines that achieve a systemic effect.

The benzodiazepines may be administered in a single dose or in multiple doses. Whereas a single dose is a particularly straightforward administration scheme, in certain cases multiple doses (preferably 2 doses, but 3, 4 or more doses also possible), are preferred. For example, a first dose may provide a certain extent of sedation to the patient that facilitates the administration of the subsequent dose(s). Similarly, administration to a pre-sedated patient (either by administration of the same benzodiazepine or a different sedative) may be preferred.

### Intranasal and intrapulmonary formulations

The present invention further relates to formulations containing the benzodiazepine. These may be used for example in therapy as described herein, in the OINDPs of the invention and the devices of the invention. Formulations according to the present invention describe compositions containing the benzodiazepine suitable for intranasal and intrapulmonary administration, respectively. The formulations can be liquid solutions, liquid dispersions, liquid emulsions or solid preparations. The formulations as described herein are additionally to be understood to further characterize the benzodiazepine for medical use (e.g. the benzodiazepine for use by intrapulmonary and/or intranasal administration of the benzodiazepine or salt) and the benzodiazepine contained in the composition, OINDP and/or the device of the invention.

According to the present invention the preferred formulations are aqueous and contain a carrier and/or at least one excipient, in particular at least one substance selected from the group of a mucoadhesive, a permeability enhancer, a co-solvent, a solubility enhancer and a permeability enhancer. Preferred substances are described further below and preferably include a polymer, preferably a polysaccharide, a polyether, a dextrin and a organosulfur compound. The most preferred polysaccharachides are polyaminosaccharides, in particular chitosan. The most preferred polyether are polyethylene glycols (PEG), in particular PEG having a molecular weight of 200 to 2000, especially PEG 400 (MW = 380 to 420, in particular 400 g/mol). The most preferred dextrins are cyclodextrins, preferably sulfoalkyl ether cyclodextrins, in particular sulfobutylether betacyclodextrin (i.e. captisol). The most preferred organosulfur compound is dimethyl sulfoxide (DMSO). Besides these substances further preferred substances according to the invention are alcohols, in particular ethanol and propylene glycol, and glycofurol. These substances may serve as carrier and/or excipient and therefore may have properties that are preferred in the context of the invention.

When propylene glycol is contained in the formulation, its amount therein is preferably 2 to 40 %, preferably 5 to 30 %, most preferably 10 to 20 % relative to the total amount of the formulation (percentages in v/v).

When glycofurol is contained in the formulation, its amount therein is preferably 2 to 40 %, preferably 5 to 30 %, most preferably 10 to 20 % relative to the total amount of the formulation (percentages in v/v).

When captisol is contained in the formulation, its amount therein is preferably 2 to 40 %, preferably 5 to 30 %, most preferably 10 to 20 % relative to the total amount of the formulation (percentages in w/v in case of a liquid formulation and w/w in case of a solid formulation).

When chitosan is contained in the formulation, its amount therein is preferably 0.1 to 5 %, preferably, 0.2 to 3 %, most preferably 0.5 to 1.5 %, in particular about 0.5 to about 1.0 % relative to the total amount of the formulation (percentages in w/v in case of a liquid formulation and w/w in case of a solid formulation).

When PEG 400 (molecular weight about 380 to 420 g/mol) is contained in the formulation, its amount therein is preferably 1 to 40 %, preferably 5 to 30 %, more preferably 8 to 25 %, most preferably 10 to 20 %, in particular 10 % relative to the total amount of the formulation (percentages in w/v in case of a liquid formulation and w/w in case of a solid formulation).

When DMSO is contained in the formulation, its amount therein is preferably 10 to 100 %, preferably 20 to 100 %, more preferably 20 to 80 %, in particular 20 to 50 %. These amounts are particular useful for intranasal formulations. For intrapulmonary formulations, DMSO in amounts of 2 to 20 %, preferably 5 to 15 %, more preferably 8 to 12 %, most preferably about 10 % relative to the total amount of the formulation are preferred (percentages in v/v).

When ethanol is contained in the formulation, its amount therein is preferably 0.5 to 30 %, preferably 1 to 20 %, more preferably 5 to 15 %, most preferably about 10 relative to the total amount of the formulation (percentages in v/v).

### Vehicle

Formulations comprising the benzodiazepines according to the invention may contain a vehicle. According to the invention the term "vehicle" is defined as a substance added to the drug substance as a medium for conveying the active ingredient. The vehicle preferably does not have any pharmacological properties in the quantity used.

The vehicle may be gaseous or liquid. In case it is liquid the vehicle is preferably aqueous. A particularly preferred vehicle for intranasal formulations comprises or consists of water, a combination of water and polyethylene glycol or a combination of water and chitosan as further described in the following. In the context of intrapulmonary formulations, the vehicle may comprise or consist of a propellant.

### Carrier

Formulations comprising the benzodiazepines according to the invention may contain a carrier. Carriers are substances that may serve to deliver the drug to the target. In a preferred embodiment, the carrier is particulate. Suitable carriers in the context of the invention are microparticles and nanoparticles, dendrimers, micellae, emulsions, liposomes, mucoadhesives, dextrins, saccharides and polymers. The present invention is not restricted to any of said carriers; however, mucoadhesives and dextrins are particularly preferred.

Mucoadhesives describe a substance or a system which attaches to a mucosal surface. They may improve drug absorption. The mucoadhesive is for example alginate or cellulose. Preferred mucoadhesives in the context of the invention are polysaccharides, preferably amino polysaccharides, more preferably chitosan, hyaluronic acid or heparin, most preferably chitosan. Other preferred mucoadhesives are polyethers, preferably polyethylene glycol.

In one embodiment, liposomes are included in the formulations comprising the benzodiazepine of the invention. Liposomes are phospholipid vesicles composed of lipid bilayers enclosing one or more aqueous compartments in which the benzodiazepine and, if present, other substances are included.

In a preferred embodiment, microparticles or nanoparticles are included in the formulations comprising the benzodiazepine of the invention. In addition to delivering the drug to its target they may also provide a prolonged residence time with the nasal mucosa and thus enhance absorption. Microparticles are solid particles with diameters ranging from about 1 to about 100µm. Nanoparticles are solid colloidal particles with diameters ranging from about 1 to about 1000 nm. The preferred diameter is 50 to 300 nm. They comprise macromolecular materials, in which the benzodiazepine is dissolved, entrapped, encapsulated, adsorbed and/or chemically attached. Suitable materials include cyclodextrins such as, beta-cyclodextrin, gamma-cyclodextrin, and methylated cyclodextrins. Preferred materials of micro- and nanoparticles are polymers such as polyethers, polylactic acid, polyisobutylcynoacrylate, hydroxypropyl cellulose, hydroxypropyl methyl cellulose, starch, albumin, dextran, alginate, gellan and gelatin. Particularly preferred are polyethers, in particular polyethylene glycol (PEG). When PEG is used its molecular weight is preferably 400 g/mol and its amount is preferably as defined above, i.e. 1 to 40 %, preferably 5 to 30 %, more preferably 8 to 25 %, most preferably 10 to 20 %, in particular 10 % relative to the total amount of the formulation.

Other preferred materials are muco-adhesive polymers, in particular polysaccharides, preferably amino polysaccharides, more preferably chitosan, hyaluronic acid or heparin, most preferably chitosan. The chitosan most preferred chosen for delivery of the benzodiazepines has a mean molecular weight of 10 to 1000 kDa, preferably 50 to 500 kDa, most preferably about 200 kDa. The degree of deacetylation is preferably 50 to 100 %, in particular 80 to 90%. In the formulations, chitosan is preferably used in an amount as defined above, i.e. 0.1 to 5 %, preferably, 0.2 to 3 %, most preferably 0.5 to 1.5 %, in particular about 0.5 to about 1.0 % relative to the total amount of the formulation.

### Excipients

Formulations comprising the benzodiazepines according to the invention may contain one or more excipients. According to the invention the term "excipient" is defined as an ingredient added intentionally to the drug substance which should not have pharmacological properties in the quantity used. Such excipients can provide some other beneficial purpose be this to aid processing, dissolution, drug delivery via the target route of administration or aid stability. Suitable excipients in the context of the invention are diluents, solubilizers, antioxidants, preservatives, buffering agents, surfactants, agents that increase viscosity, flavoring agents, humectants and absorption enhancers. If one or more excipients are added, the employed quantity preferably does not irritate the nasal or pulmonary mucosa after single or repeated administration.

### Diluents

A diluent suitable for administration to the nasal mucosa may be included in the intranasal formulation, if the intranasal formulation is liquid. Suitable diluents include aqueous and non-aqueous diluents, and combinations thereof. Exemplary aqueous diluents are saline, water or combinations thereof. Non-aqueous diluents include alcohols, particularly polyhydroxy alcohols such as glycerol, and vegetable or mineral oils or combinations thereof. In a preferred embodiment, water or aqueous solutions are used as a diluent. The diluents can be added in various concentrations and combinations to form solutions, suspensions or emulsions (oil-in-water or water-in-oil).

### Solubilizers

Aqueous solubility of the benzodiazepine may be a limitation for nasal drug delivery in solution. To enhance its solubility solvents or co-solvents such as glycols, alcohols, Transcutol (diethylene glycol monoethyl ether), medium chain glycerides and Labrasol (saturated polyglycolyzed C8-C10 glyceride) can be added to the formulation. The formulation may also comprise surfactants or cyclodextrins such as HP-β-Cyclodextrin that may serve as a biocompatible solubilizer, stabilizer and lipophilic absorption enhancer as well.

### Antioxidants

To prevent drug oxidation an antioxidant may be included in the formulation of the inventions. Commonly used antioxidants are sodium metabisulfite, potassium metabisulfite, sodium bisulfite, butylated hydroxytoluene, butylated hydroxyanisole and tocopherol.

### Preservatives

A preservative may be included in the formulations to prevent microbial growth, in particular when they are aqueous. Parabens, benzalkonium chloride, methyl, ethyl, propyl or butylparaben, phenyl ethyl alcohol, phenylethyl alcohol, benzethonium, EDTA and benzoyl alcohol are exemplary preservatives in intranasal formulations of the invention.

### PH and buffer agents

If the formulations are aqueous the pH value is preferably selected so that (i) irritation of the nasal or pulmonary mucosa is avoided; (ii) the drug is available in unionized form to allow absorption; (iii) growth of pathogenic bacteria is prevented in the nasal passage; (iv) functionality of excipients such as preservatives are maintained; and/or (v) normal ciliary movement is sustained. In the context of the intranasal administration it is therefore preferred that nasal formulations have a pH value of 3 to 9, preferably 4 to 8, more preferably 5 to 8, most preferably 6 to 8, in particular 6.5 to 7.5.

Due to the low volume that can be administered by the intranasal route nasal secretions may alter the pH of the administrated drug. In one embodiment, a buffer may be included in the formulation to avoid an alteration of the concentration of unionized drug available for absorption. The buffer capacity is selected to maintain the preferred pH or preferred pH range, in particular pH 6.5 to 7.5 in-situ. Suitable buffering agents include salts of citrate, acetate, or phosphate, for example, sodium citrate, sodium acetate, sodium phosphate, and combinations thereof. In another embodiment, no dedicated buffer agent is added to the formulation, and preferably the formulation comprises no buffer. If the formulation comprises no buffer, a pH change may occur at the site of administration (e.g. lung, nasal structures) that may result in the formation of solid benzodiazepine. In a preferred embodiment this solidification leads to an improved absorption of the benzodiazepine, in particular remimazolam.

### Surfactant

To facilitate drop or spray delivery a surfactant may be employed. Surfactants are substances that lower the surface tension between two liquids or between a liquid and a solid and may increase the solubility. The surfactants may be anionic (e.g. sodium lauryl sulphate), cationic (e.g., cetrimide), non-ionic (e.g. Tween 80, Span) or amphoteric (e.g., Lecitin, N-dodecyl alanine).

### Viscosity and viscosifying agents

The absorption of drugs is influenced by the residence time between the drug and the epithelial tissue. The mucociliary clearance is inversely related to the residence time and therefore inversely proportional to the absorption of drugs administered. To prolong the residence time of the benzodiazepine in the nasal cavity bioadhesives, microparticles or chitosan may be added to the formulation or the viscosity of the formulation may be increased. The viscosity of liquid formulations comprising the benzodiazepines of the invention, in particular the liquid formulations for intranasal administration is preferably 2 to 50 mPa*s, more preferably 5 to 20 mPa*s and most preferably 10 to 15 mPa*s.

To adjust the viscosity of the formulations a viscosifying agent may be added. A viscosifying agent is a substance that increases the viscosity of the formulation. Suitable viscosifying agents include hydroxyethylcellulose, hydroxypropylmethylcellulose, methylcellulose, carboxymethylcellulose, ethylcellulose, polyvinyl alcohol, polyvinylpyrrolidone, carboxy-vinyl polymer, carrageenan, carbopol, and combinations thereof.

### Flavoring agents

A flavoring agent may be added to the formulations of the invention to enhance the taste of the formulation, in particular formulations intended for intranasal administration. Suitable flavoring agents include vanilla (vanillin), mint, raspberry, orange, lemon, grapefruit, caramel, cherry flavors and combinations thereof.

### Humectants

Many allergic and chronic diseases are often connected with crusts and drying of mucous membrane. Thus, the formulations may comprise a humectant to provide adequate moisture, in particular when the benzodiazepine is administered as a gel. Examples of suitable humectants include glycerin, sorbitol and mannitol.

### Absorption Enhancers

The formulation may contain an absorption enhancer to improve membrane permeability and/or reduce enzymatic degradation by aminopeptidases. The absorption enhancers can be physical or chemical enhancers. Chemical enhancers act by destructing the nasal mucosa very often in an irreversible way. Physical enhancers affect nasal clearance reversibly by forming a gel. Examples of chemical enhancers are chelating agents, fatty acids, bile acid salts, surfactants, and preservatives. Preferred absorption enhancers in the context of the invention are polysaccharides, preferably amino polysaccharides, more preferably chitosan.

### Osmolarity

Drug absorption can be affected by tonicity of the formulation. To avoid shrinking of epithelial cells and inhibiting or ceasing ciliary activity isotonic or hypotonic formulations are preferred.

### Dosage, drug concentration and volume

The formulations preferably contain the benzodiazepines in an amount which is pharmaceutically effective upon intranasal or intrapulmonary administration. For example, the preferred doses of Remimazolam for intranasal and intrapulmonary administration are preferably slightly higher than Remimazolam IV and range from 5 to 250 mg, preferably 25 to 200 mg, more preferably 50 to 125 mg.

The dosage for each subject may vary, however, a preferred amount or dosage of the benzodiazepines for intranasal and intrapulmonary administration to obtain sedation or hypnosis in mammals is 0.05 to 25.0 mg/kg of body weight, and more particularly, 0.1 to 2.5 mg/kg of body weight, preferably 0.1 to 1.25 mg/kg of body weight, the above being based on the weight of the benzodiazepine. A preferred amount or dosage of the benzodiazepines for intranasal and intrapulmonary administration to obtain anxiolysis in mammals is 0.05 to 25.0 mg/kg of body weight, and more particularly, 0.1 to 2.5 mg/kg of body weight, preferably 0.1 to 1.25 mg/kg of body weight, the above being based on the weight of the benzodiazepine. A preferred amount or dosage of the benzodiazepines for intranasal and intrapulmonary administration to obtain muscle relaxation in mammals is 0.05 to 25.0 mg/kg of body weight, and more particularly, 0.1 to 2.5 mg/kg of body weight, preferably 0.1 to 1.25 mg/kg of body weight, the above being based on the weight of the benzodiazepine. A preferred amount or dosage of the benzodiazepines for intranasal and intrapulmonary administration to treat convulsions in mammals is 0.05 to 25.0 mg/kg of body weight, and more particularly, 0.1 to 2.5 mg/kg of body weight, preferably 0.1 to 1.25 mg/kg of body weight, the above being based on the weight of the benzodiazepine. The preferred dosage for humans is therefore 5 to 250 mg.

In case the intranasal formulations are liquid, the volume that can be absorbed through the nasal mucosa is limited by the area of the nasal passages. Thus, for a reproducible dose response, the volumes should ideally not exceed about 200 µL (100 µL into each nostril). The volumes of liquid formulations for intranasal administration thus preferably is 25 µL to 600 µL, preferably 25 µL to 300 µL, more preferably 50 µL to 150 µL.

A preferred liquid formulation for intranasal administration contains 1 to 1000 mg/ml, preferably 25 to 800 mg/mL, more preferably 50 to 500 mg/mL of the benzodiazepine of the present invention, in particular remimazolam. A preferred intrapulmonary formulation contains 5 to 250 mg, preferably 25 to 200 mg, more preferably 50 to 125 mg of the benzodiazepine, in particular remimazolam.

The most preferred formulation is a solid composition, preferably a lyophilized solid composition, in particular as described in WO 2013/174883 A1. Preferably, the lyophilized solid composition comprises remimazolam and lactose in a weight ratio of 1:13. Most preferably, remimazolam is contained in said composition in an amount of 26 mg. In a preferred embodiment, this composition comprises a mucoadhesive as described above and is reconstituted prior to administration or the solid composition is directly used as a powder drug product.

### Preparation of the formulations

The formulations comprising the benzodiazepine of the invention can be made, for example, by mixing the benzodiazepine, for example a benzodiazepine contained in a composition which is in the solid state, preferably a lyophilized solid composition as described in WO 2013/174883 A1 (see also above) and, if present, the vehicle, carrier and/or one or more excipients at, for example, room temperature under aseptic conditions to form a mixture. Conveniently, the mixture is filtered, for example, by a 0.22 micron filter. It will be understood that the order of mixing is not critical. In preferred embodiments, the formulations are sterile.

To prepare dry powder formulations the formulation are preferably dried by spray drying. Spray drying may produce respirable colloidal particles in the solid state. In this method, the feed solution is supplied at room temperature and pumped to the nozzle where it is atomized by the nozzle gas. The dispersed solution is then dried by preheated drying gas in a special chamber to remove water moisture from the system, thus forming dry particles. This method produces typically particles of above 2-µm size and advantageously results in uniform particle morphology.

Alternatively, dry powder formulations may preferably be prepared by spray freeze drying. This method combines spray-drying and freeze-drying. It involves spraying the drug solution into liquid nitrogen as a freezing medium followed by lyophilization. This method usually produces light and porous particles and high fine particle fraction.

Supercritical fluid technology is another preferred method to produce dry powder formulations. Hereby, small particles are obtained from a dispersion in supercritical fluids, such as supercritical carbon dioxide by controlled crystallization of the drug. This method can be used for production of microparticles, nanoparticles and liposomes.

Further suitable methods for obtaining particulates are solvent precipitation, double emulsion/solvent evaporation and particle replication in non-wetting templates (PRINT).

### Dosage forms for intranasal and intrapulmonary administration

Dosage forms of medicaments intended for intranasal and intrapulmonary administration are preferably a liquid, a suspension or a solid. A suspension is a liquid preparation containing solid particles dispersed in a liquid vehicle. The dosage forms are preferably metered. For examples, metered drops/sprays mean that the dispenser that includes the drops/spray delivers the drops/spray containing a metered dose (a predetermined quantity) of the benzodiazepine.

One preferred dosage form in the context of the intranasal administration route includes nasal drops. Nasal drops are simple to self-administer and enjoy wide acceptance among patients including children. Drops are deposited mostly in the posterior portion of the nose and thus removed rapidly into the nasal pharynx. A concern with drops is often how to precisely control the drug's dose which is particularly important for the administration of benzodiazepines. Reproducible dose spending means should therefore be ensured.

Another intranasal dosage form by which the benzodiazepines of the invention can be administered is nasal sprays. Nasal sprays typically contain the benzodiazepine dissolved or suspended in a solution or a mixture of excipients (e.g. preservatives, viscosity modifiers, emulsifiers, buffering agents) in a non-pressurized dispenser. Nasal sprays have several advantages including compactness of the delivery device, convenience, simplicity of use, and accuracy of delivering dosages of 25 to 200 µL. They are deposited in the anterior portion of the nose and cleared slowly into nasal pharynx by mucociliary clearance. The nasal spray as used herein can be a liquid or a suspension.

Another intranasal dosage form is a nasal aerosol. Nasal aerosols differ from nasal sprays by the method of drug dispensing: in aerosols, a drug is dispensed due to an excess of pressure and releases through a valve. In sprays, a drug is dispensed due to forcing away by a micropump bucket, while the pressure in the vial is similar to atmosphere pressure. Aerosols have similar advantages as sprays.

The benzodiazepines of the invention may alternatively preferably be administered by nasal emulsions, ointments, gels, pastes or creams. These are highly viscous solutions or suspensions applied to the nasal mucosa. Their efficacy of drug absorption may be better as compared to drops because the high viscosity may prevent the benzodiazepine from running out of the nasopharynx.

Due to the limited volume of drug that can be efficiently delivered to the nasal mucosa, liquid intranasal dosage forms usually have higher concentrations as the corresponding IV dosage forms. When substances become poorly soluble or are instable in liquid form, powders can be used to administer the benzodiazepines of the invention. Further advantages of powders are that they do not require preservatives and have usually a higher stability as compared to liquid formulations. The main limitation on intranasal powder application is related to its irritating effect on the nasal mucosa.

One dosage form in context of intrapulmonary administration is an inhalation aerosol. Inhalation aerosols are usually packaged under pressure and contain the benzodiazepine which is released upon activation of a valve system into the respiratory tract, in particular the lungs. The released aerosol is a colloid of fine solid particles (suspension) or liquid droplets (solution) in air or another gas. Accordingly, the aerosol may be a solution or a suspension aerosol. The liquid droplets or solid particles have preferably a diameter of less than 100 µm, more preferably less than 10 µm, most preferably less than 1 µm.

Another dosage form in context of intrapulmonary administration is inhalation sprays. Inhalation sprays are typically aqueous based and do not contain any propellant. They deliver the benzodiazepine to the lungs by oral inhalation.

Nebulized inhalation solutions and suspensions may also be used to deliver the benzodiazepine by the intrapulmonary route. Nebulized inhalation solutions and suspensions are typically aqueous-based formulations that contain the benzodiazepine. The nebulized inhalation solutions and suspensions deliver the benzodiazepine to the lungs by oral inhalation for systemic effects and are used with a nebulizer.

Dry powder inhalation is an alternative to aerosol inhalation. The medicament is usually included in a capsule for manual loading or within the inhaler. Dry powders are typically delivered by an inhaler to the lungs by oral inhalation. The dry powders as used herein can be formulated neat. Neat formulations contain the drug alone or quasi-alone e.g. as spry dried powder. The dry powders as used herein can be also formulated with a carrier such as lactose.

Intrapulmonary dosage forms are preferably metered, i.e. are delivered to the lungs in a predetermined quantity.

### Devices for intranasal and intrapulmonary delivery

Devices for intranasal delivery in the context of the present invention include spray pump systems, pipettes for delivering drops, metered-dose spray pumps, nasal pressurized metered-dose inhalers, powder spray systems, breath-actuated powder inhalers and nasal powder insufflators. The intranasal delivery device may be filled with a single dose amount or a multi-dose amount of the intranasal formulation.

Using the intrapulmonary route the benzodiazepine may be administered with a metered dose inhaler. A metered-dose inhaler (MDI) provides a fine mist of medicament, generally with an aerodynamic particle size of less than 5 µm.

Dry powder inhalers can be alternatively used to deliver the benzodiazepine intrapulmonary. Dry powder inhalers present powders as single-dose or multidose powders.

Another device for intrapulmonary delivery is a nebulizer including ultrasonic and air jet nebulizers. In ultrasonic nebulizers, ultrasound waves are formed in an ultrasonic nebulizer chamber by a ceramic piezoelectric crystal that vibrates when electrically excited. This generates an aerosol cloud at the solution surface. The aerosol produced by an air jet nebulizer is generated when compressed air is forced through an orifice. A liquid may be withdrawn from a perpendicular nozzle (the Bernoulli Effect) to mix with the air jet which is atomized using baffles to facilitate the formation of the aerosol cloud.

### Co-administration

A further active ingredient may be co-administered with the benzodiazepine, either in a single formulation with the benzodiazepine or in separate formulations. Co-administration may augment the pharmaceutical effect of the benzodiazepine or the pharmaceutical effect of the co-administered active ingredient. In the context of the invention it is preferred to co-administer (simultaneously or sequentially, preferably sequentially) an analgesic.

The analgesic is preferably an opioid. This term refers to compounds which have the same mode of action as the constituents of opium, the dried milky liquid of the poppy seed, *Papaver somniferum.* All opioid drugs interact in biological systems with the same type of receptor, the so called opioid receptor. According to the analgesia and side effect profile five types of opioid receptors, the µ-receptor (ligand = morphine), the κ[kappa]-receptor (ligand = ketazocine), the delta-receptor (ligand = deltorphine II), the σ[sigma]-receptor (ligand = SKF 10081), as well as the later-identified ORL1 - receptor (ligand = nociceptin) are known. Corresponding to other receptor systems, binding studies as well as functional investigations indicate that subtypes of opioid receptors exist. Within the µ- and δ-receptor type 2 subtypes, the µ-1 and µ-2 and δ-1 and δ -2 have been described. The κ-receptor contains an additional κ-3 subtype. Especially in regards to the µ-opioid receptor its two subtypes are included for the purposes of this invention.

The opioid is preferably selected from the group consisting of:
- morphine, codeine, thebain, papaverin, narcotine,
- dihydrocodeine, thebacon, anileridine, piminodine, phenoperidine, furethidine, [alpha]-prodin, trimeperidine, profadol, methadone, levomethadyl acetate, phenadoxone, dipipanone, themalon, N-methylmorphinan, dextrometorphane, phenazocine, ketocyclazocine, bremazocine, carfentanil, fentanyl, lofentanil, ohmefentanil, pitramide, benztriamide, loperamide, U-50488, 1-benzyl-4-(4-bromo-phenyl)-4-dimethylamino-cyclohexanol;
- alfentanil, buprenorphine, butorphanol, dextromoramide, dextropropoxyphene, dezocine, diamorphine, diphenoxylate, ethylmorphine, etorphine, hydrocodone, hydromorphone, ketobemidone, levomethadone, levomethadyl-acetate, levorphanol, meptazinol, nalbuphine, nalorphine, oxycodone, oxymorphone, pentazocine, pethidine, piritramide, remifentanil, sufentanil, tilidine, tramadol, tapentadol,
- met-enkephalin, leu-enkephalin, nociceptin, β-endorphin, endomorphin-1, endomorphin-2, metorphamid, dynorphin-A, dynorphin-B, and α-neoendorphin.

Fentanyls, in particular fentanyl, alfentanil, sufentanil and remifentanil are particularly preferred co-administered agents.

### The benzodiazepines of the invention

According to the invention the benzodiazepine is generally a compound according to formula (I) 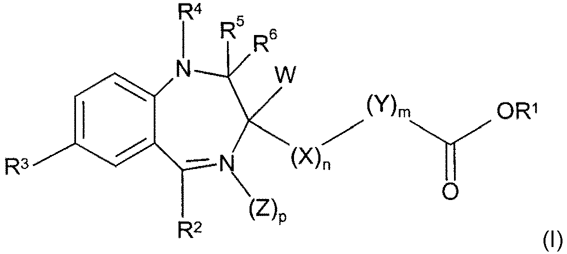 wherein
W is H, a C₁-C₄ branched alkyl, or a straight chained alkyl;
X is CH₂, NH, or NCH₃; n is 1 or 2;
Y is O or CH₂; m is 0 or 1;
Z is O;
R¹ is a C₁-C₇ straight chain alkyl, a C₃-C₇ branched chain alkyl, a C₁-C₄ haloalkyl, a C₃-C₇ cycloalkyl, an aryl, a heteroaryl, an aralkyl, or a heteroaralkyl;
R² is phenyl, 2-halophenyl or 2-pyridyl,
R³ is H, Cl, Br, F, I, CF₃, or NO₂;
(1) R⁴ is H, a C₁-C₄ alkyl, or a dialkylaminoalkyl and R⁵ and R⁶ together represent a single oxygen or S atom which is linked to the diazepine ring by a double bond and p is zero or 1; or (2) R⁴ and R⁵ together form a double bond in the diazepine ring and R⁶ represents the group NHR⁷ wherein R⁷ is H, C₁₋₄ alkyl, C₁₋₄ hydroxyalkyl, benzyl or benzyl mono or disubstituted independently with halogen substituents, C₁₋₄ alkylpyridyl or C₁₋₄ alkylimidazolyl and p is zero; or (3) R⁴, R⁵ and R⁶ form the group -CR⁸=U-V= wherein R⁸ is hydrogen, C₁₋₄ alkyl or C₁₋₃ hydroxyalkyl, U is N or CR⁹ wherein R⁹ is H, C₁₋₄ alkyl, C₁₋₃ hydroxyalkyl or C₁₋₄ alkoxy-C₁₋₄alkyl, V is N or CH and p is zero.

The term "aryl", alone or in combination, is defined herein as a monocyclic or polycyclic group, preferably a monocyclic or bicyclic group, e.g., phenyl or naphthyl, which can be unsubstituted or substituted, for example, with one or more and, in particular, one to three substituents selected from halogen, C₁₋₄ branched or straight chained alkyl, C₁₋₄ alkoxy, C₁₋₄ haloalkyl, hydroxy, nitro, amino, and the like. The term "heteroaryl" is defined herein as a 5-membered or 6-membered heterocyclic aromatic group which can optionally carry a fused benzene ring and wherein said 5-membered or 6-membered heterocyclic aromatic group can be unsubstituted or substituted, for example, with one or more and, in particular, one to three substituents selected from halogen, C₁₋₄ branched or straight chained alkyl, C₁₋₄ alkoxy, C₁₋₄ haloalkyl, hydroxy, nitro, amino, and the like. The term "alkoxy", alone or in combination, is defined herein to include an alkyl group, which is attached through an oxygen atom to the parent molecular subunit. Exemplary alkoxy groups include but are not necessarily limited to methoxy, ethoxy and isopropoxy. The term "aralkyl" is defined herein as an alkyl group, in which one of the hydrogen atoms is replaced by an aryl group. The term "heteroaralkyl" is defined herein as an alkyl group, in which one of the hydrogen atoms is replaced by a heteroaryl group.

Exemplary branched or straight chained C₁₋₄ alkyl groups include but are not necessarily limited to methyl, ethyl, propyl, isopropyl, isobutyl and n-butyl. Exemplary C₁₇ straight chain alkyl groups include, but are not necessarily limited to, methyl, ethyl, propyl, n-butyl, n-hexyl and n-heptyl. Exemplary C₃₋₇ branched chain alkyl groups include, but are not necessarily limited to, isopropyl, isobutyl, sec-butyl, tert-butyl, isopentyl, neopentyl, tert-pentyl and isohexyl. Exemplary C₃₋₇ cycloalkyl groups include, but are not necessarily limited to, cyclopropyl, cyclobutyl, cyclopentyl, cyclohexyl and cycloheptyl. Exemplary C₁₋₄ haloalkyl groups include, but are not necessarily limited, to methyl, ethyl, propyl, isopropyl, isobutyl and n-butyl substituted independently with one or more halogens, e.g., fluoro, chloro, bromo and iodo.

The compounds of formula (I) where the groups R⁴ and R⁵ and R⁶ together form the group -CR⁸=U-V= and p is 0 represent a preferred embodiment of the invention and may be conveniently represented by the compound of formula (II): 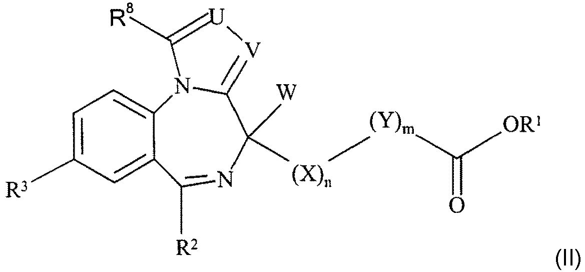 wherein R¹, R², R³, R³, U, V, W, X, Y, n and m have the meanings given for formula (I).

Further preferred are compounds of formula (I) 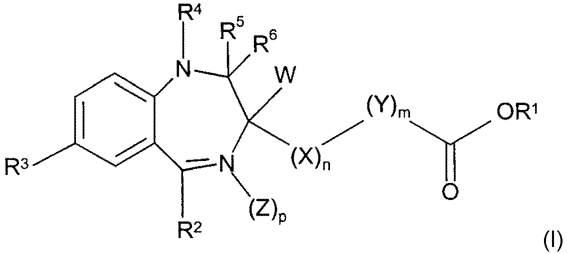 with
W is H;
X is CH₂; n is 1;
Y is CH₂; m is 1;
Z is O; p is 0 or 1;
R¹ is CH₃, CH₂CH₃, CH₂CH₂CH₃, CH(CH₃)₂ or CH₂CH(CH₃)₂
R² is 2-fluorohenyl, 2-chlorophenyl or 2-pyridyl;
R³ is Cl or Br;
(1) R⁴ is H, a C₁-C₄ alkyl, or a dialkylaminoalkyl and R⁵ and R⁶ together represent a single oxygen or S atom which is linked to the diazepine ring by a double bond and p is zero or 1; or (2) R⁴ and R⁵ together is a double bond in the diazepine ring and R⁶ represents the group NHR⁷ wherein R⁷ is H, C₁₋₄ alkyl, C₁₋₄ hydroxyalkyl, benzyl or benzyl mono or disubstituted independently with halogen substituents, C₁₋₄ alkylpyridyl or C₁₋₄ alkylmidazolyl and p is zero; or (3) R⁴, R⁵ and R⁶ form the group-CR⁸=U-V= wherein R³ is hydrogen, C₁₋₄ alkyl or C₁₋₃ hydroxyalkyl, U is N or CR⁹ wherein R⁹ is H, C₁₋₄ alkyl, C₁₋₃ hydroxyalkyl or C₁₋₄ alkoxy, V is N or CH and p is zero.

Preferably, in particular in compounds according to formula (II), W is H, X is CH₂, n is 1; Y is CH₂, m is 1; R¹ is CH₃, CH₂CH₃, CH₂CH₂CH₃, CH(CH₃)₂ or CH₂CH(CH₃)₂; R² is 2-fluorophenyl, 2-chlorophenyl or 2-pyridyl; R³ is Cl or Br; R³ is H, CH₃ or CH₂OH; R⁹ is H, CH₃, CH₂OH or CH₂O-t-butyl; U is CR⁹ or N; and V is N or CH.

Particularly preferred amongst these compounds are compounds according to formula (II), wherein in each compound W is H, X is CH₂, n is 1, Y is CH₂, m is 1 and wherein R¹, R², R³, R⁸, U and V for each compound are as follows:

| R¹ | R² | R³ | R⁸ | U | | V |
|---|---|---|---|---|---|---|
| CH₃ | 2-fluorophenyl | Cl | H | CH | | N |
| CH₃ | 2-fluorophenyl | Cl | CH₃ | CH | | N |
| CH₃ | 2-fluorophenyl | Cl | H | C-CH₃ | | N |
| CH₃ | 2-fluorophenyl | Cl | H | C-CH₂OH | | N |
| CH₃ | 2-fluorophenyl | Cl | CH₂OH | CH | | N |
| CH₃ | 2-pyridyl | Cl | H | CH | | N |
| CH₃ | 2-pyridyl | Cl | CH₃ | CH | | N |
| CH₃ | 2-pyridyl | Br | CH₃ | CH | | N |
| CH₃ | 2-pyridyl | Br | H | C-CH₃ | | N |
| CH₃ | 2-pyridyl | Cl | H | C-CH₃ | | N |
| CH₃ | 2-pyridyl | Cl | H | C-CH₂OH | | N |
| CH₃ | 2-pyridyl | Cl | CH₂OH | CH | | N |
| CH₃ | 2-pyridyl | Cl | CH₃ | C-CH₃ | | N |
| CH₃ | 2-chlorophenyl | Cl | CH₃ | N | | N |
| CH₃ | 2-fluorophenyl | Cl | CH₃ | N | | N |
| CH₃ | 2-fluorophenyl | Cl | CH₃ | N | | N |
| CH₃ | 2-fluorophenyl | Cl | H | N | | CH |
| CH₃ | 2-fluorophenyl | Cl | CH₃ | N | | CH |
| CH₃ | 2-fluorophenyl | Cl | H | C-CH₂O-t-butyl | | N |
| CH₃ | 2-pyridyl | Cl | CH₃ | C-CH₂OH | | N |

Amongst these compounds the most preferred is remimazolam (INN), wherein W is H, X is CH₂, n is 1, Y is CH₂, m is 1, R¹ is CH₃, R² is 2-pyridyl, R³ is Br, R³ is CH₃, U is CH and V is N. According to IUPAC system remimazolam is methyl 3-[(4S)-8-bromo-1-methyl-6-(pyridin-2-yl)-4H-imidazo[1,2-a][1,4]benzodiazepin-4-yl]propanoate. It is clinically developed by PAION AG, Aachen under the internal designation "CNS7056". The besylate form of CNS7056 is also called "CNS7056B".

The benzodiazepines and in particular remimazolam are preferably produced according to the methods described in WO 00/69836 A1, in particular according to the method comprising (a) preparing 3-[(S)-7-bromo-2-oxo-5-pyridin-2-yl-2,3-dihydro-1 H-1 ,4-benzodiazepin- 3-yl]-propionic acid methyl ester by reacting (2-amino-5-bromophenyl)-pyridin-2-yl-methanone in chloroform with an alpha-Fmoc-protected-amino acid chloride (obtained by reacting FMOC-Glu(OMe)-OH and oxalylchloride in dichloromethane), treating the obtained amide with triethylamine in dichloromethane, then with acetic acid in 1 ,2- dichloroethane, isolating the compound of formula (D), and (b) reacting the compound of formula (D) with a suspension of sodium hydride in THF, treating the reaction mixture with bis-morpholinophosphochloridate (BPMC) in THF, filtering the reaction mixture, reacting the filtrate with DL-1-amino-propanol, purifying the alcoholic adduct obtained, treating that purified alcoholic adduct with a mixture of DMSO and oxalyl chloride in dichloromethane, treating the reaction mixture with triethylamine, diluting with ethyl acetate, washing with aqueous solutions and concentrating to give a foam, treating that foam with a catalytic amount of p-toluenesulfonic acid, neutralizing the solution with sodium hydrogenocarbonate and isolating Remimazolam.

Alternatively, the benzodiazepines and in particular remimazolam are preferably produced according to the methods described in WO 2008/007071, namely by adding benzene sulfonic acid to a solution of that compound in toluene or ethyl acetate, stirring, filtering, washing with toluene or ethyl acetate and drying under vacuum. That method yields 3-[(4S)-8-Bromo-1-methyl-6-(2-pyridinyl)-4H-imidazo[1,2-a][1,4] benzodiazepine-4-yl]propionic acid methyl ester benzene sulfonate.

Another preferred production method for Remimazolam is disclosed in WO 2011/032692 A1. The method comprises reacting 3-[(S)-7-bromo-2-((R and/or S)-2-hydroxy-propylamino)-5-pyridin-2-yl-3H-benzo[e][1,4]diazepin-3-yl]-propionic acid methyl ester of formula (EM) 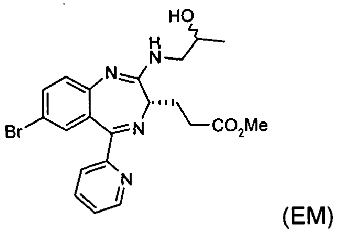 with an oxidizing agent which is a hypervalent iodine compound of formula (DM) 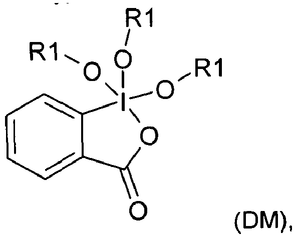 wherein R1 is acyl, such as 1 ,1 ,1-triacetoxy-1 ,1-dihydro-1 ,2-benzoiodoxol-3(1 H)-one (Dess-Martin periodinane).

Another preferred method according to which Remimazolam can be produced is disclosed in WO 2014/136730A1. The method includes subjecting, to an oxidation reaction, a compound selected from the group consisting of 3-[(S)-7-bromo-2-(2-hydroxypropylamino)-5-pyridin-2-yl-3H-benzo[e][1,4]diazepin-3-yl]propionic acid methyl ester, 3-[(S)-7-bromo-2-((R)-2-hydroxy-propylamino)-5-pyridin-2-yl-3H-benzo[e][1,4]diazepin-3-yl]propionic acid methyl ester, and 3-[(S)-7-bromo-2-((S)-2-hydroxy-propylamino)-5-pyridin-2-yl-3H-benzo[e][1,4]diazepin-3-yl]propionic acid methyl ester in the presence of at least one oxidation catalyst.

Compounds according to formula (I) and (II) possess a stereocenter. According to the invention enantiomeric pure forms can be used, which are substantially free of the other enantiomer, but also racemic mixtures can be used.

The composition according to the invention might comprise the free form of the benzodiazepine, but in a preferred embodiment of the invention the benzodiazepine is used in the form of a salt, in particular in the form of an inorganic or organic salt. In a very preferred embodiment the benzodiazepine is used in the salt in a cationic form.

The counter ion of the cationic benzodiazepine is preferably selected from halogenides, in particular fluoride, chloride or bromide, sulfate, organic sulfates, sulfonate, organic sulfonates, nitrate, phosphate, salicylate, tartrate, citrate, maleate, formiate, malonate, succinate, isethionate, lactobionate and sulfamate.

The salts of the invention are obtained by reaction of the benzodiazepine with suitable acids, in particular by reaction with the following acids: hydrochloric, hydrobromic, sulfuric, nitric, phosphoric, salicylic, p-toluenesulfonic, tartaric, citric, methanesulfonic, maleic, formic, malonic, succinic, isethionic, lactobionic, naphtalene-2-sulfonic, sulfamic, ethanesulfonic and benzenesulfonic.

In a preferred embodiment the counter ion is selected from organic sulfates and organic sulfonates, in particular from aromatic sulfates and aromatic sulfonates. In a very preferred embodiment an organic sulfonate is used as counter ion, preferably an aromatic sulfonate, in particular p-toluenesulfonic acid (tosylate), naphthalene-2-sulfonic acid, ethanesulfonic acid (esylate) or benzenesulfonic acid, wherein benzenesulfonic acid (besylate) is the most preferred counter ion.

The most preferred salts according to the invention are the besylate salt (as disclosed in WO 2008/007071 A1) or the esylate salt (as disclosed in WO 2008/007081 A1) of remimazolam. The tosylate of remimazolam is also preferred and is subject matter of WO 2013/029431 A1.

The formulations, in particular powder-based formulations according to the invention may comprise at least one pharmaceutically acceptable hygroscopic excipient as defined in WO 2013/174883, preferably a disaccharide, in particular one which is selected among dextran, lactose, maltose, sucrose and trehalose. The disaccharides (preferably lactose, in particular lactose monohydrate) can be combined with the dextran (preferably dextran 40), preferably in a lyophilized formulation. The pharmaceutically acceptable hygroscopic excipient is especially suitable in order to prepare stable solid formulations - e.g. lyophilized or spray dried compositions - for benzodiazepines, in particular remimazolam salts, which have a favourable reconstitution time. A formulation with the above listed disaccharide/dextran mixture is preferably lyophilized and further preferably comprises remimazolam, either in its besylate, esylate or tosylate salt. Especially preferred is the besylate salt.

Alternatively, a formulation free from hygroscopic excipients may be preferred in certain embodiments, in order to facilitate the handling and application of the formulation. This may be especially preferred when higher amounts are to be administered, such as greater than or equal to 20 mg, 25 mg, 30 mg, 35 mg, 40 mg, 50 mg, 75 mg, 100 mg, 125 mg, 150 mg, 200 mg or 250 mg.

In the most preferred embodiment of the invention the benzodiazepine salt is remimazolam besylate. When the remimazolam besylate is crystalline, the crystalline polymorph is preferably besylate Form 1, besylate Form 2, besylate Form 3 or besylate Form 4 as defined in WO 2013/174883. The besylate Forms 1 to 4 may be prepared and crystallised by using the methods and solvents disclosed in WO 2008/007071 A1. A preferred salt is the besylate Form 1 or Form 2 (Form 2 being particularly preferred) based on the robustness of formation, yield, purity and chemical and solid form stability. In one embodiment of the invention the composition comprises a mixture of Forms 1, 2, 3 and 4. However compositions with only one of the Forms 1 to 4 are preferred.

In another preferred embodiment of the invention the benzodiazepine salt is remimazolam esylate. When the remimazolam esylate is crystalline, the crystalline polymorph is preferably esylate Form 1 or esylate Form 2 as defined in WO 2013/174883. The esylate Forms 1 and 2 may be prepared and crystallised by using the methods and solvents disclosed in WO 2008/007081 A1. A preferred salt is the esylate Form 1 based on the robustness of formation, yield, purity and chemical and solid form stability. In one embodiment of the invention the composition comprises a mixture of Forms 1, and 2. However compositions with only one of the Forms 1 or 2 are preferred.

For storage the formulations may be lyophilized or spray dried as described in WO 2013/174883. The solid form of the compositions, in particular the lyophilized or spray dried solids, show very good storage stability, in particular at storage conditions of 40°C/75%RH.

The present invention also provides a method for producing sedation or hypnosis in a mammal, which comprises administering to the mammal an effective sedative or hypnotic amount of a pharmaceutical of the present invention as hereinbefore defined. The present invention also provides a method for inducing anxiolysis in a mammal, which comprises administering to the mammal an effective anxiolytic amount of a pharmaceutical of the present invention as hereinbefore defined. The present invention also provides a method for inducing muscle relaxation in a mammal, which comprises administering to the mammal an effective muscle relaxant amount of a pharmaceutical of the present invention as hereinbefore defined. The present invention also provides a method for treating convulsions in a mammal, which comprises administering to the mammal an effective anticonvulsant amount of a pharmaceutical of the present invention as hereinbefore defined. The present invention also provides a method for inducing or maintaining anesthesia in a mammal, which comprises administering to the mammal an effective anesthetic amount of a pharmaceutical of the present invention as hereinbefore defined.

The present invention also provides the use of a sedative or hypnotic amount of a composition of the present invention as hereinbefore defined in the manufacture of a medicament for producing sedation or hypnosis in a mammal, including in a human. The present invention also provides the use of an anxiolytic amount of a composition of the present invention as hereinbefore defined in the manufacture of a medicament for producing anxiolysis in a mammal, including in a human. The present invention also provides the use of a muscle relaxant amount of a composition of the present invention as hereinbefore defined in the manufacture of a medicament for producing muscle relaxation in a mammal, including in a human. The present invention also provides the use of an anticonvulsant amount of a composition of the present invention as hereinbefore defined in the manufacture of a medicament for treating convulsions in a mammal, including in a human. The present invention also provides the use of an anesthetic amount of a composition of the present invention as hereinbefore defined in the manufacture of a medicament for inducing or maintaining anesthesia in a mammal, including in a human.

The present invention also provides the use of a pharmaceutical according to the invention for producing sedation or hypnosis and/or inducing anxiolysis and/or inducing muscle relaxation and/or treating convulsions and/or inducing or maintaining anaesthesia in a mammal.

Due to the simplicity and/or painlessness that may be achieved, the medicament of the invention is particularly suited for infants (up to 12 months of age), children (1 to 12 years of age), and adolescents (12 to 17 years of age). As in particular children often fear injections, the preferred patients to be treated with the benzodiazepines of the invention are children and infants, for example children undergoing a diagnostic or surgical procedure; children prior to receiving intravenous anaesthesia or inhalational anaesthesia. Also of particular interest are patients for whom an intravenous access is difficult or impossible; or patients which suffer from a panic attack or epilepsy. Moreover, medical personnel are not required for administering the drug. Neither are hygienic conditions or disposal of needles a concern. The present invention thus allows for self-administration of the medicament by non-skilled persons without risks of low patient compliance.

A particular embodiment of the invention relates to remimazolam, in particular remimazolam besylate, remimazolam tosylate or remimazolam esylate, formulated in an aqueous composition comprising a polyether, in particular polyethylene glycol (PEG) (MW = 400 g/mol), which is present in an amount of 10 to 20 % by weight, in particular 10 % by weight relative to the total volume of the formulation and its use for intranasal administration. The formulation has preferably a viscosity of 10 to 15 mPa*s and/or a pH value of 6 to 8.

Another particular embodiment of the invention relates to remimazolam, in particular remimazolam besylate, remimazolam tosylate or remimazolam esylate, formulated in an aqueous composition comprising a polyether, in particular polyethylene glycol (PEG) (MW = 400 g/mol), which is present in an amount of 10 to 20 % by weight, in particular 10 % by weight relative to the total volume of the formulation and its use for intrapulmonary administration. The formulation has preferably a viscosity of 10 to 15 mPa*s and/or a pH value of 6 to 8.

A further particular embodiment of the invention relates to remimazolam, in particular remimazolam besylate, remimazolam tosylate or remimazolam esylate, formulated in an aqueous composition comprising a polysaccharide, preferably an amino polysaccharide, more preferably chitosan (MW = 50 - 500 kDa), which is present in an amount of 0.2 to 3 % by weight, in particular 0.5 to 1.5 % by weight relative to the total volume of the formulation and its use for intranasal administration. The formulation has preferably a viscosity of 10 to 15 mPa*s and/or a pH value of 6 to 8.

A yet further particular embodiment of the invention relates to remimazolam, in particular remimazolam besylate, remimazolam tosylate or remimazolam esylate, formulated in an aqueous composition comprising a polysaccharide, preferably an amino polysaccharide, more preferably chitosan (MW = 50 - 500 kDa), which is present in an amount of 0.2 to 3 % by weight, in particular 0.5 to 1.5 % by weight relative to the total volume of the formulation and its use for intrapulmonary administration. The formulation has preferably a viscosity of 10 to 15 mPa*s and/or a pH value of 6 to 8.

### EXAMPLES

### Example 1: Intranasal Remimazolam

The objective of this study was to determine the maximum tolerated or maximum feasible dose (MTD) of remimazolam, a short acting sedative/anaesthetic, when given in various vehicles *via* the intranasal route to male rats. Part A: Escalating dose phase; four ascending doses until MTD was reached (with a 3-4 day washout between each dose level). Part B: Fixed dose phase; the MTD determined in part A was dosed by the intranasal and intravenous routes for 3 days. During Part B, the toxicokinetic characteristics of remimazolam were determined.

### 1. Experimental Design

**Text Table 1**

| Experimental Design | | | | |
|---|---|---|---|---|
| Part A - MTD Phase (Intranasal Route - Four Ascending Doses) | | | | |
| **Group No.** | **Test Item** | **Target Dosage Levels (mg/kg)** | **Dosage Volume (mL)** | **Dosage Concentration (mg/mL)** |
| 6 | Remimazolam in Water for Injection (WFI) | 2,4,8,16 | 0.1 | 5, 10, 20, 40 |
| 7 | Remimazolam in WFI with 1% Chitosan | 2,4,8,16 | 0.1 | 5, 10, 20, 40 |
| 8 | Remimazolam in WFI with 20% PEG | 2,4,8,16 | 0.1 | 5, 10, 20, 40 |

**Part B - Fixed Dose Phase (At MTD Phase Maximum Dose)**

| **Group No.** | **Test Item** | **Dose Route** | **Target Dosage Level (mg/kg)** | **Dosage Volume (mL)** | **Dosage Concentration (mg/mL)** |
|---|---|---|---|---|---|
| 1 | Vehicle (WFI) | Intranasal | 0 | 0.1 | 0 |
| 2 | Remimazolam in WFI | Intranasal | 16 | 0.1 | 40 |
| 3 | Remimazolam in WFI with 1% Chitosan | Intranasal | 16 | 0.1 | 40 |
| 4 | Remimazolam in WFI with 20% PEG | Intranasal | 16 | 0.1 | 40 |
| 5 | Remimazolam in WFI | IV Injection | 16 | 0.1 | 40 |

The test formulations and control item were administered to animals (Groups 6 to 8, part A and Groups 1 to 4 part B only) by once daily intranasal instillation. The doses were given using a micropipette with attached appropriately sized plastic tip. All control and dosed animals received 2 instillations (25µL) into each nostril (of the vehicle control or the test formulations) on each treatment day (for a total volume of 0.1 mL). During dosing, the rats were held with their head in a vertical position. The micropipette was kept ca. 0.5 mm into the first nostril and the drop of formulation (25µL) was instilled. Immediately afterwards the second nostril was instilled. The animal was kept vertical for a few seconds to allow the formulation to disappear into the nose and then the procedure was repeated for both nostrils, when dosing was complete the animal was then put back in its cage. The first day of dosing was designated as Day 1. Each dosing formulation container was inverted prior to initiation of dosing.

In Part B, Group 5 animals received an intravenous injection once daily for 3 days. Intravenous (bolus) injection (*via* the lateral tail vein) was delivered using a sterile needle and a disposable syringe. The site of dose administration was cleaned with sterile wipes before dose administration.

### 2. Procedures, Observations, and Measurements

The ascending dose phase (Part A) was concluded with only recoverable clinical observations and no effect on bodyweight at the highest solubility for each vehicle.

The fixed dose phase (Part B) was staggered to start over two consecutive days (to accommodate Irwin observations). On the first of these two days, 3 animals from Groups 1 to 4 were dosed and then observed and on the second day the remaining animals were dosed and observed at as close as possible to the same time of day as they were dosed on the first day.

For all animals bodyweights were collected daily and standard clinical observations were recorded at appropriate intervals prior to the Irwin testing as below.

Animals were observed during the Irwin testing at the following timepoints: Predose, +15 mins, + 30 mins by an observer blinded to the treatment of the animals.

The following parameters were included in the observations:
- Occurrence of vocalization, stereotypies, aggressiveness, abnormal gait, straub tail, tremor, twitches, convulsions, body posture, sedation, catalepsy, ptosis, exophthalmos, salivation, lacrimation, piloerection, abnormal respiration, defecation urination and death.
- Increase or decrease of spontaneous activity, touch response, and body tonus.

Increase of sniffing, grooming, scratching and rearing.
- Pupil size was measured using a guidance chart to estimate the size in millimeters.
- Decreased pinna reflex, traction response and grip strength. Any additional symptoms observed such as abnormal respiration, defecation and urination were also required to be noted.
- Frequencies of animals exhibiting symptoms were recorded. Symptoms were scored from 0 to 3, where 0 represents no finding and 3 the highest score.
- Body temperature was measured by using a probe inserted approx. 2 cm past the anal sphincter.

The remaining dose phase sampling and endpoints are described in the tables below. The "Day" in each table refers to the day of dosing, staggered, as described above.

**Text Table 2**

| Blood Sample Collection Schedule for Pharmacokinetic Analysis | | | | | | |
|---|---|---|---|---|---|---|
| **Group No.** | **No. of Males** | **Sample Collection Time Points (Time Postdose) on Day 3** | | | | |
| | | **1 min** | **3 min** | **5 min** | **15 min** | **30 min** |
| 1 | 3 | X | - | - | - | - |
| | 3 | - | X | - | - | - |
| 2 | 3 | - | X | - | X | - |
| | 3 | X | - | X | - | X |
| 3 | 3 | - | X | - | X | - |
| | 3 | X | - | X | - | X |
| 4 | 3 | - | X | - | X | - |
| | 3 | X | - | X | - | X |
| 5 | 3 | - | X | - | X | - |
| | 3 | X | - | X | - | X |

| | | | | | | |
|---|---|---|---|---|---|---|
| x = sample collected; - = not applicable. | | | | | | |

**Text Table 3**

| Terminal Procedures | | | | | | | |
|---|---|---|---|---|---|---|---|
| **Group Number** | **Number of Animals** | **Scheduled Euthanasia Day** | **Necropsy Procedures** | | | **Histology** | **Histo-pathology** |
| | **M** | | **Necropsy** | **Tissue Collection^{a}** | **Organ Weights^{a}** | | |
| 1 | 6^{b} | 4 | X | X | - | X | X |
| 2 | 6^{b} | | | | | X | X |
| 3 | 6^{b} | | | | | X | X |
| 4 | 6^{b} | | | | | X | X |
| 5 | 6^{b} | | | | | - | - |

| | | | | | | | |
|---|---|---|---|---|---|---|---|
| X = procedure conducted; - = not applicable ^{a} See below for details of the sampling. | | | | | | | |

**Text Table 4**

| Respiratory Tract Tissue Collection and Preservation | | | | |
|---|---|---|---|---|
| **Tissues** | **Weigh** | **Collect** | **Microscopic Evaluation** | **Comment** |
| Animal Identification | - | X | - | - |
| Nasal cavity (Fig. 6) | - | X | X | After dissection from the carcass, the nasal cavity was gently flushed with 10% neutral buffered formalin in order to ensure removal of air pockets from within the nasal cavity. Decalcification was undertaken. Four transverse sections of the nasal cavity were produced and evaluated. Sections were taken as follows (see Fig. 6): |
| | | | | Section I - cross sectioned 2-3mm posterior to the upper incisor teeth. |
| | | | | Section II - cross sectioned through the first palatal ridge. |
| | | | | Section III - cross sectioned through the middle of the first upper molar teeth, passed through the anterior (medial) portion of both eye orbits. |
| | | | | Section IV - cross sectioned through the third upper molar teeth. |
| | | | | One level included the nasopharyngeal duct and the Nasal Associated Lymphoid Tissue (NALT). |

### 3. RESULTS

### a) Mortality

There were no unscheduled deaths on this study.

### b) Clinical Observations

Observations included rolling gait, subdued behaviour, laboured breathing, decreased activity and occasional loss of righting reflex, along with signs related to the dosing procedure (sneezing and red discharge nose). In Part A the observations were present more frequently on each subsequent day of dosing, in response to the ascending dose. In Part B there were no major differences between the intranasal groups dosed with remimazolam.

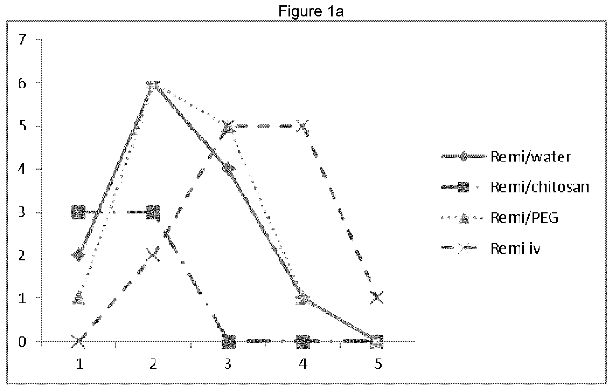

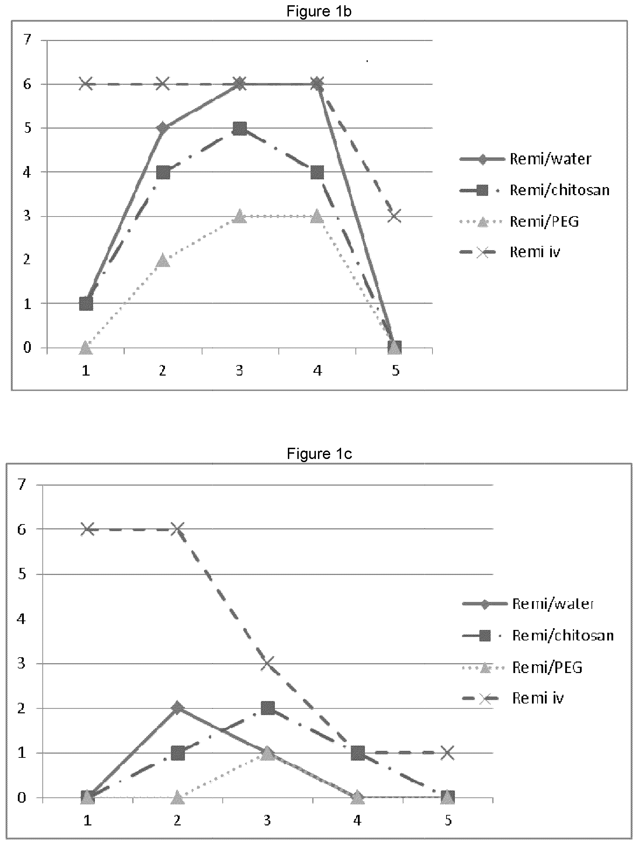

Text Table 5 shows the results and Figure 1 illustrates observations with regard to rolling gait, subdued behaviour and decreased activity. "Remi" stands for remimazolam. The number of animals showing a particular clinical observation is indicated in each group.

**Text Table 5**

| Clinical observations | | | | | | |
|---|---|---|---|---|---|---|
| | **Group** | **IPD** | **5 min** | **10 min** | **15 min** | **30 min** |
| **rolling gait** | Remi/water | 2 | 6 | 4 | 1 | 0 |
| | Remi/chitosan | 3 | 3 | 0 | 0 | 0 |
| | Remi/PEG | 1 | 6 | 5 | 1 | 0 |
| | Remi IV | 0 | 2 | 5 | 5 | 1 |
| **decreased activity** | Remi/water | 0 | 2 | 1 | 0 | 0 |
| | Remi/chitosan | 0 | 1 | 2 | 1 | 0 |
| | Remi/PEG | 0 | 0 | 1 | 0 | 0 |
| | Remi iv | 6 | 6 | 3 | 1 | 1 |
| **subdued behavior** | Remi/water | 1 | 5 | 6 | 6 | 0 |
| | Remi/chitosan | 1 | 4 | 5 | 4 | 0 |
| | Remi/PEG | 0 | 2 | 3 | 3 | 0 |
| | Remi iv | 6 | 6 | 6 | 6 | 3 |
| **loss of righting reflex** | Remi/water | 0 | 0 | 0 | 0 | 0 |
| | Remi/chitosan | 0 | 0 | 0 | 0 | 0 |
| | Remi/PEG | 0 | 0 | 0 | 0 | 0 |
| | Remi iv | 6 | 0 | 0 | 0 | 0 |
| IPD = immediately post dose | | | | | | |

### c) Body Weights and Body Weight Changes

Bodyweights were unaffected by any of the formulations during the single ascending doses or during the repeat dose phase at the maximum feasible dose.

### d) Irwin Observations

There were no significant differences at the 15 and 30 minute timepoints between animals treated via the intranasal route with the different formulations.

### e) Body Temperatures

Body temperatures were unaffected by any of the formulations at the 15 and 30 minute timepoints during the Irwin screening.

### f) Effect of Remimazolam on Pupil Size (mm)

Pupil sizes were unaffected by any of the formulations at the 15 and 30 minute timepoints during the Irwin screening.

### g) Gross Pathology

No test item-related gross findings were noted. The gross findings observed were considered incidental, of the nature commonly observed in this strain and age of rat, and, therefore, were considered unrelated to administration of remimazolam.

### h) Histopathology

Minimal transitional epithelial metaplasia was observed in the ventral meatus of the anterior nasal cavity in one male treated with remimazolam in water (Group 2). One animal had minimal inflammation of the olfactory epithelium.

Minimal or mild transitional epithelial metaplasia, sometimes confined to the ventral meatus, was observed in the anterior nasal cavity of all males treated with remimazolam in water with 1% Chitosan (Group 3). Two animals also had minimal crust of the olfactory epithelium.

There were no microscopic findings associated with administration of remimazolam in water with 20% PEG (Group 4).

Test item-related microscopic findings are summarized in Text Table 6.

**Text Table 6**

| Summary Microscopic Findings - Scheduled Euthanasia Animals (Day 4) | | | | |
|---|---|---|---|---|
| | **Males** | | | |
| **Group** | **1** | **2** | **3** | **4** |
| **Dose (mg/kg)** | **0** | **16** | **16** | **16** |
| **No. animals examined** | **6** | **6** | **6** | **6** |
| **Nasal Cavity (No. Examined)** | (6) | (6) | (6) | (6) |
| Metaplasia, transitional epithelium | 0 | 1 | 6 | 0 |
| Minimal | 0 | 1 | 4 | 0 |
| Mild | 0 | 0 | 2 | 0 |
| Inflammation, olfactory epithelium, minimal | 0 | 1 | 0 | 0 |
| Crust, olfactory epithelium, minimal | 0 | 0 | 2 | 0 |

No other microscopic findings were observed in the nasal cavity.

### i) Pharmacokinetic analysis: Plasma levels of Remimazolam and its main metabolite CNS7054

**Text Table 7**

| Pharmacokinetics for CNS7054 (carboxylic acid metabolite of CNS7056) and Remimazolam | | | | | |
|---|---|---|---|---|---|
| **Group** | **1 min** | **3 min** | **5 min** | **15 min** | **30 min** |
| | **CNS7054 [ng/ml]** | | | | |
| 2 (Remi Water) | 1270 | 6680 | 8757 | 1301 | 1358 |
| 3 (Remi Chitosan) | 2273 | 1627 | 4296 | 6360 | 1124 |
| 4 (Remi PEG) | 3703 | 3274 | 1203 | 524 | 562 |
| 5 (Remi IV) | 26210 | 15045 | 20952 | 10962 | 7387 |

| | **Remimazolam [ng/ml]** | | | | |
|---|---|---|---|---|---|
| 2 (Remi Water) | 41,6 | 49,7 | 219,4 | 0 | 0 |
| 3 (Remi Chitosan) | 52,6 | 10,1 | 36,4 | 0 | 0 |
| 4 (Remi PEG) | 268,7 | 53,0 | 0 | 0 | 0 |
| 5 (Remi IV) | 31,4 | 0 | 0 | 0 | 0 |

The results indicate that following intranasal administration, rats are systemically exposed to remimazolam and its main metabolite CNS7054.

### 4. DISCUSSION

The Group 3 formulation (with chitosan) had a lower pH than the other formulations (as hydrochloric acid was included in the formulation in order to allow the chitosan to fully dissolve) and this lower pH is considered likely to be partially responsible for the increased severity of the histopathological findings and the crusting response compared to Group 2 (with water, only).

All formulations were viscous. The Group 4 formulation (with PEG) was notably the most viscous.

All clinical observations considered to be due to intranasal dosing with remimazolam (rolling gait, subdued behaviour, laboured breathing, decreased activity and occasional loss of righting reflex) were present up to 15 min following dosing at which point in time the plasma levels of remimazolam have returned to 0 for all dosing groups, including intravenous administration.

### 5. CONCLUSION

In conclusion, intranasal administration of remimazolam was well tolerated in rats, animals showed good recovery after each dosing session and presented as clinically healthy prior to necropsy.

When compared with the intravenous route the intranasal route was efficacious, but not as efficacious in eliciting clinical observations consistent with the pharmacological effect for the same amount of test item.

The histopathology observed for Group 3 is considered to be undesirable in terms of repeated administration.

### Example 2: Intrapulmonary remimazolam

This experiment confirms the feasibility of delivering remimazolam as an adjunct to remifentanil via inhalation.

### 1. Methods

Rats were exposed to remimazolam and remifentanil aerosol alone and in combination. Analgesia was quantified by using a tail flick meter and pulmonary injury was assessed using mechanics measurements.

Time to tail flick study was performed using male Sprague-Dawley rats weighing between 200-300g. Pulmonary mechanics measurements were performed using 8-week-old male C57BI/6 mice weighing 19-25 grams and a Flexivent FX-1 instrument (Scireq, Montreal, Qc, Canada).

**Inhalation Chamber:** The whole body inhalation chamber used was as described in Bevans et al. (Bevans T, Deering-Rice CE, Stockmann C, Light AR, Reilly CA, Sakata D. Inhaled remifentanil in Rodents. Anesthesia & Analgesia:ln Press.), but with an additional integrated Aerogen Lab ultrasonic nebulizer generously provided by Aerogen Ltd. (Galway, Ireland). The aerogen nebulizer produces 2.5-4.0 volume mean diameter aerosolized particles and was used to nebulize remimazolam. **Analgesic Testing:** Analgesia was assessed as in Bevans et al (see supra). Briefly, time to tail flick was measured using a IITC Tail Flick Analgesia Meter. Tails were tested 2 cm from the tip using 50% light intensity and a pre-programmed cut-off time of 20 s to prevent tissue damage or surface burn injury to the rat.

**Time to Tail Flick Study:** This study of 25 rats was performed in addition to the dose response study of 53 rats already performed using inhaled remifentanil (see Bevans et al supra). Time to tail flick in drug-exposed groups was compared to time to tail flick in pre-test baseline and inhaled saline control groups. For this study, remimazolam was tested at 10 and 25 mg/mL, and in combination with remifentanil 100 mcg/mL or 250 mcg/mL.

**Pulmonary Mechanics:** 30 mice (n=5/group) were used to assess pulmonary function following acute aerosolized remimazolam exposure and acute and repeated exposure to combined inhaled remimazolam and remifentanil using *Flexi-Vent* FX-1 small animal ventilator (Scireq, Montreal, Qc, Canada). Specifically measured were changes in lung resistance (Rrs), airway resistance (Rn), tissue resistance (G), lung compliance (Crs), lung elastance (Ers) and tissue elastance (H). These were determined using a constant-phase model which has been extensively used to assess lung mechanics in mice (e.g., Irvin CG, Bates JHT. Measuring the lung function in the mouse: the challenge of size. Respir Res 2003;4:4). Methods were also as previously described in Bevans et al. (see supra).

For acute remimazolam exposure, control mice were exposed to vehicle (10% DMSO/ 90% normal saline) for 5 treatments followed by a methacholine challenge of 25 mg/mL. Treatment mice were exposed to one dose of vehicle followed by four treatments of increasing concentrations of remimazolam (5, 10, 15, 20 mg/mL), followed by a methacholine challenge (25 mg/mL). For combination exposures, mice were exposed to vehicle control, followed by 4 treatments of 200 mcg/mL remifentanil combined with 20 mg/mL remimazolam, followed by a methacholine challenge (25 mg/mL). These mice were compared to mice exposed 5 times to vehicle followed by methacholine. For repeated sub-acute exposure, mice were exposed to a combination of 250 mcg/mL remifentanil and 20 mg/mL remimazolam every other day for 3 treatments via the whole body exposure chamber. Forty-eight hours following the third exposure, pulmonary mechanics were measured as above.

**Statistical Analysis:** Statistical analysis was performed as in Bevans et al (see supra). The experiments featured in this study were powered to achieve 80% power with one-way ANOVA and two-sample t-tests. The Shapiro-Wilk test was used to assess the normality of the data and/or the residuals prior to performing any statistical comparisons. Data are expressed as medians (interquartile range [IQR]) and comparisons between groups at a single point in time were performed using the t test or the non-parametric Mann-Whitney U test, as appropriate.

The time to tail flick test was performed using a Student's t test. The acute pulmonary mechanics experiments were performed using a one-way ANOVA. For all comparisons, *p*<0.001 was considered to be statistically significant. All statistical comparisons were two-sided. R 3.1.1 (R Foundation for Statistical Computing, Vienna, Austria) and Graphpad Prism (La Jolla, CA, USA) were used to perform the power calculations and statistical analyses.

### 2. Results

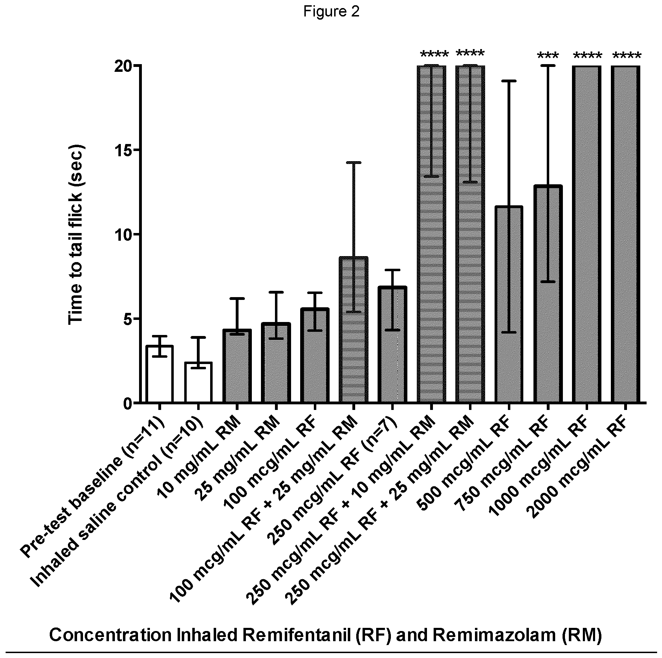

**Time to Tail Flick:** Inhalation of remimazolam alone failed to produce analgesia. Concentrations >25 mg/mL could not be tested due to lack of solubility in a reasonable vehicle. When remimazolam (10 or 25 mg/mL) was administered in combination with 250 mcg/mL remifentanil there was a significant difference in time to tail flick (P<0.0001), comparable to analgesia achieved using 1000 mcg/mL remifentanil alone (P<0.0001) (Fig 2).

**Pulmonary Mechanics:** Acute inhalation delivery of remimazolam up to 20 mg/mL did not alter the pulmonary mechanics of mice (Fig 3). Likewise, mice acutely (Fig 4) or sub-acutely (Fig 5) exposed to a combination of remifentanil and remimazolam showed no alterations to pulmonary mechanics, except when comparing the methacholine challenge for airway resistance, where sub-acutely exposed mice showed diminished changes in lung resistance compared to vehicle exposed mice (P<0.0007). These data show that remimazolam alone or in combination with remifentanil does not cause lung irritation, bronchospasm, or other adverse pulmonary events. The decrease in lung resistance is attributable to remifentanil.

### 3. Discussion

This study show that remimazolam, when administered in conjunction with remifentanil, has a synergistic effect on analgesia while also sharing the desired pharmacokinetic, pharmacodynamic, and safety profile of ester-based, short acting agents. Remimazolam is therefore considered to enter the systemic blood circulation in therapeutically active configuration.

### Example 3: Solubility screen

Various vehicles were used to test the solubilization of remimazolam drug product (a lyophilized solid composition comprising 20 mg remimazolam and lactose in a weight ratio of 1:13).The vehicles included WFI (water for injection), 1 % w/v Chitosan in WFI and 20 % w/v PEG 400 in WFI.

The vehicles were prepared as follows:
- WFI as obtained;
- 1% w/v Chitosan: Chitosan was weighed out (1 g for 100 mL volume) and added to WFI to about 80% of the final volume, then stirred using a magnetic stirrer bar. The pH was adjusted with 1 M HCl until the Chitosan was dissolved; this required about 5 mL for a total volume of 100 mL. Once fully dissolved the solution was transferred to a 100 mL volumetric flask and made to volume with WFI. The solution was returned to the beaker and stirred to mix.
- 20 % w/v PEG 400 in WFI: 20 g PEG 400 was weighed directly into a glass beaker to which WFI was added to a volume of about 60% (i.e. about 60 mL WFI). The solution was stirred using a magnetic stirrer bar until fully dissolved and then transferred to a 100 mL volumetric flask and made to volume with WFI (100 mL). The solution was returned to the beaker and stirred to mix.

The vehicles were tested regarding their capacity to reconstitute 20 mg remimazolam in 0.4 mL vehicle and the solutions' stability. The results are as follows:
A volume of 0.4 mL of all three vehicles successfully reconstituted the remimazolam drug product. The 20% w/v PEG 400 solution was stable for at least 6 hours (when reconstituted with 0.4 mL and at least 24 hours when reconstituted with 0.5 mL) compared to less than 2 hours for WFI and 1% w/v Chitosan in WFI.

### Example 4: Study of CNS7056 (Remimazolam) in Göttingen minipigs following single intranasal administration

The aim of the study was to obtain information on the absorption and pharmacokinetics, the sedation profile and the local tolerability (evaluated by rhinoscopy) of Remimazolam in minipigs following a single intranasal administration of the non-reconstituted formulated drug product which is a lyophilisate containing the besylate salt of remimazolam as active ingredient and dextran 40 and lactose monohydrate as excipients. The minipig was selected because of the similarity of anatomic conditions of the nose with those of humans.

### Conduct of study

Test item: Remimazolam (CNS7056), Batch no. TT284
Formulated drug product: 33.2% lactose monohydrate, 49.7% dextran 40, 17.1% remimazolam besylate
One vial contains: 50 mg Remimazolam (active ingredient) or 69.37 mg Remimazolam besylate
Vehicle: Not applicable.
Species and strain: Göttingen minipigs, non-naïve
Supplier: Ellegaard Göttingen Minipigs A/S, Denmark
Number and sex of animals: 3 female animals, animal numbers 1 to 3
Body weight (at dosing): 20.2 to 25.0 kg
Age (at dosing); 1 - 2 years
Adaptation period: 2 weeks
Diet: The animals were fed with a suitable amount according to their age and body weight (as recommended by the breeder Ellegaard, DK).
Drinking water: offered ad libitum
Dose level: 25 mg Remimazolam (active ingredient) per animal

Route of administration: Intranasal administration via spatula into the left nostril, the right nostril remained untreated and served as control.

Administration frequency and duration: Single administration on test day 1 followed by 3 off-dose days
Administration amount: 25 mg/animal/day. The total amount of 25 mg Remimazolam (active ingredient) per animal was divided into 4 portions of 3 x 5 mg and 1 x 10 mg. The 4 portions per animal were weighed in shortly before administration and administered into the left nostril within 3 to 5 minutes. The first portion was approx. 5 mg Remimazolam. The test item was administered as the dry formulated Clinical Trial Material (lyophilisate), i.e. no reconstitution was carried out. Prior to administration the lyophilisate was ground into a powder. The consistency of the test item was pasty upon contact with the moisture of the nasal mucosa. Therefore, the application procedure needed special attention.

### Rationale for dose selection

The dose levels were selected based on toxicological data and available data obtained from an internal pilot study in Göttingen minipigs for feasibility: This exploratory study had revealed that the amount of drug product applicable as a powder is limited by its strong hygroscopy (confirmed in the present study). Therefore, for the sake of controllable handling and to keep the administration procedure short, it was decided to administer 25 mg of drug product.

The Remimazolam (lyophilisate) was applied using a spatula in small portions at short intervals (total application time of 25 mg Remimazolam per animal: 3 to 5 minutes).

The application of the bulk of the drug product powder was possible after administration of a first fraction of the total dose, which was at risk of being partially snorted out, though. Following the administration of the first fraction, however, the intranasal positioning of the remaining fractions was better tolerated. The risk of snorting in pre-sedated animals was only minimal (Also the two minipigs that later showed normal responses to stimuli seemed to experience some level of relaxation after the first portion). However, the administered material tended to stick to the nasal orifice, partly occluding the entrance.

### Findings

Clinical signs: No test item-related changes in behavior (except for sedation, see below) or external appearance were observed after intranasal administration of 25 mg Remimazolam/animal.

Mortality: None of the animals died during the course of the study.

Body weight: No test item-related influence on the body weight was observed.

Food and drinking water consumption: No test item-related influence was noted.

Sedative effect score: One of three animals (animal no. 2) revealed moderate (3) and marked (4) sedative effects for showing no reaction to acoustic and light stimulus, respectively, (Symptom 1), and moderate (3) sedative effects for mechanical stimulus (Symptom 2). The scoring of sedative effects following intranasal administration is summarized in the following table:

| **General sedation while the animals were placed in the hammock** | | **Score** |
|---|---|---|
| **Symptom 1 ^{a)}** | **Symptom 2 ^{b)}** | |
| Normal | Normal | 0 (none) |
| Slight latency in response to acoustic stimulus | Slight latency in response to mechanical stimulus | 1 (minimal) |
| Marked latency in response to acoustic stimulus | Moderate latency in response to mechanical stimulus | 2 (mild) |
| No response to acoustic stimulus | Marked latency in response to mechanical stimulus | 3 (moderate) |
| No response to direct stimulus (light)^{#} | No response to mechanical stimulus | 4 (marked) |

| | | |
|---|---|---|
| ^{a)} Response when a sound was made by tapping on the side of the hammock/pen frame caudal to the animal's head. ^{b)} Response when the pad of the forelimb or hind limb was pinched. ^{#} If the animal did not react to the background stimulus (sound), the blink reflex was checked. | | |

Test item-related sedative effects (Symptoms 1 and 2) were noted in animal no. 2 after intranasal administration of 25 mg Remimazolam/animal starting 5 minutes after administration lasting up to 30 minutes with a maximum at 10 minutes p.a.

The maximum sedative effect score was 3 and 4 for showing no reaction to acoustic and light stimulus, respectively (Symptom 1), and 3 for mechanical stimulus (Symptom 2).

The animal nos. 1 and 3 were not affected (scores of zero were recorded).

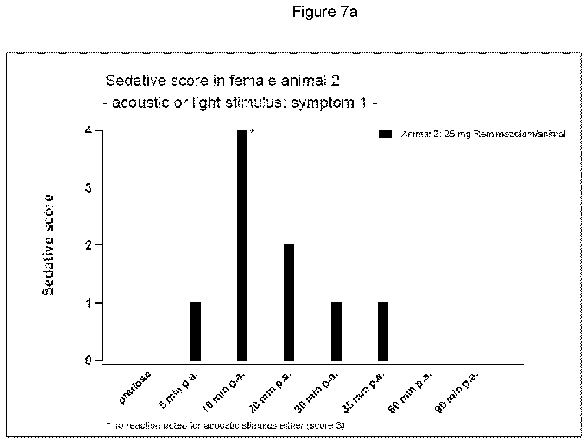

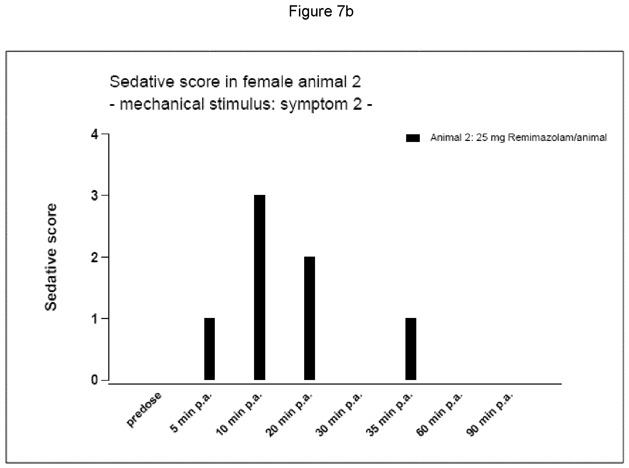

The results are given graphically in Figure 7a (Symptom 1) and Figure 7b (Symptom 2) and shown in the following table.

| **CNS7056 (Remimazolam) Sedative Effect Score** | | | | | | | | |
|---|---|---|---|---|---|---|---|---|
| Animal no. | Pre-dose | Minutes p.a. | | | | | | |
| | | 5 | 10 | 20 | 30 | 35 | 60 | 90 |
| | | **Symptom 1** | | | | | | |
| 1 f | 0 | 0 | 0 | 0 | 0 | 0 | 0 | 0 |
| 2 f | 0 | 1 | 3,4 | 2 | 1 | 1 | 0 | 0 |
| 3 f | 0 | 0 | 0 | 0 | 0 | 0 | 0 | 0 |

| | | **Symptom 2** | | | | | | |
|---|---|---|---|---|---|---|---|---|
| 1 f | 0 | 0 | 0 | 0 | 0 | 0 | 0 | 0 |
| 2 f | 0 | 1 | 3 | 2 | 0 | 1 | 0 | 0 |
| 3 f | 0 | 0 | 0 | 0 | 0 | 0 | 0 | 0 |

Rhinoscopy: No test item-related changes of the nasal mucosa in form of erythema, eschar or oedema formation were observed in any animal after nasal administration of 25 mg Remimazolam/animal.

Plasma analysis: The plasma levels of Remimazolam (CNS7056) and the metabolite CNS7054 were below the lower limit of quantification of the GLP-validated bioanalytical method (20 ng/mL CNS7056, 100 ng/mL CNS7054) in all samples except of the sample obtained from animal no. 2 at 0.25 hours p.a. in which Remimazolam was found at 50.4 ng/mL. Concentrations of Remimazolam and CNS7054 below the LLOQ of the GLP method were determined using a scientifically sound and reliable extrapolation approach.

Pharmacokinetics: The pharmacokinetic evaluation of Remimazolam (CNS7056) and its metabolite CNS7054 based on values measured within the range of the GLP bioanalytical method was not feasible as almost all plasma values were below the lower limit of quantification. An estimation of the absorption based on plasma concentrations determined by quantification in the low concentration range using a non-validated though reliable, scientifically sound extrapolation of the validated method was performed.

### Blood sampling for toxicokinetics

In order to obtain at least 500 µL EDTA/NaF plasma containing Paraoxon esterase inhibitor per animal and sampling time, blood was collected from the *vena jugularis* of all animals at the following time points. Pre-chilled tubes with a maximum volume capacity of 1200 µL were used and filled up with blood:

| **Blood sampling on test day** | **Animal numbers** | **Sampling times** | **Number of plasma samples** |
|---|---|---|---|
| 1 | 1 - 3 | - 3 min p.a. | 5 x 3 |
| | | - 15 min p.a. | |
| | | - 30 min p.a. | |
| | | - 60 min p.a. | |
| | | - 90 min p.a. | |
| | | Thereafter in 30-min intervals up to 1 h after the last sedation symptoms had been subsided | 2 x 3 |
| | | - 2.0 h p.a. | |
| | | - 2.5 h p.a. | |
| | | No observation was performed thereafter as the last sedation symptoms occurred 35 minutes after administration | |
| **Total number of samples:** | | | **21** |

| | | | |
|---|---|---|---|
| p.a.: post dosing relating to the time after intranasal administration of the last portion | | | |

### Plasma sample preparation

Paraoxon Dilution: 100 µL of Paraoxon (Paraoxon-ethyl, Sigmar-Aldrich, batch no. SZBD172XV) were added to 400 µL of acetonitrile (ACN) and mixed by inversion. All blood samples were collected into pre-chilled tubes with a maximum volume capacity of 1200 µL containing EDTA/NaF plus 1 µL of Paraoxon/ACN solution per 100 µL blood, i.e. 12 µL of Paraoxon/ACN solution per tube. The blood samples were cooled using a Cooling-Rack system (Nalgene^{®} Labtop cooler) until centrifugation. After centrifugation the plasma samples were immediately frozen and stored at ≤-15°C.

The pharmacokinetic evaluation of Remimazolam (CNS7056) and its metabolite CNS7054 was hampered by the fact that almost all plasma values were below the lower limit of quantification (LLOQ_{CNS7056}: 20 ng/mL; LLOQ_{CNS7054}: 100 ng/mL). Absorption characteristics were assessed based on bioanalytical data obtained by extrapolation of the GLP-validated method into the LOQ range.

The area under the plasma concentration curve of Remimazolam from 3 minutes to 2.5 hours after intranasal administration was estimated for the mean plasma profile using the linear trapezoidal method.

An estimation of the absorption basing on plasma concentrations determined by quantification in the low concentration range using a non-validated though reliable extrapolation of the validated method was performed.

Rationale for consideration of sub-LLQ values:
Sub-LLOQ values indicated that absorption of Remimazolam after intranasal administration of the drug product powder was definitively present. This was characterised based on plasma concentration data deduced from the HPLC-MS response by extrapolation to the zero point of values found below the lower limit of the validated method. This extrapolation was deemed to deliver a reliable estimate of the actual analyte concentrations because of minimal background noise (as shown by HPLC-MS profiles) and the close similarity of the algorithms applied for the back calculation of concentrations within the validated and the non-validated range.

### PK parameters

The Cₘₐₓ of Remimazolam achieved in animal no. 2 was 50.4 ng/mL, i.e. the only value found within the GLP-validated range. A slightly different result was obtained for the evaluation of this sample using the slightly different algorithm suitable for estimations in the concentration range below the lower limit of quantification of the GLP bioanalytical method. Cₘₐₓ in animal no. 1 was 17.4 ng/mL. Cₘₐₓ in animal no. 3 did not exceed 8.6 ng/mL and occurred relatively late after administration.

The tₘₐₓ of plasma Remimazolam was 15 min after intranasal administration in two of three animals; for the metabolite, it was 15 min in animal no. 2 and 60 min in animal nos. 1 and 3.

The AUC_{0->last} of the metabolite was similar in the three minipigs ranging between 38 and 51 ng*h/mL whereas the AUC_{0->last} of Remimazolam showed a wider range, from 7 (animal no. 3) to ~20 ng*h/mL (animal no. 2).

### Discussion

Ratios of AUC (CNS7054/Remimazolam) were 3.7, 1.9, and 6.6 for animal nos. 1 to 3, indicating a less extensive metabolism in animal no. 2. This is consistent with further pharmacokinetic details. Overall, it is obvious (and likely in view of the anatomical setting) that major portions (animal no. 1) or almost all (animal no. 3) of the intranasal dose have been sniffed in and swallowed shortly after administration, so that the observed profile likely reflects a composite of intestinal and intranasal absorption. In animal no. 3, the lag of Cmax of the metabolite and the low Remimazolam concentration profile in animal no. 3 suggest that oral absorption followed by hepatic first pass metabolism was the predominant fate of the intranasally administered dose in this animal. Animal no. 1 showed a similar pattern (although plasma concentrations of Remimazolam were somewhat higher), so that animal no. 2 probably comes closest to reflecting pharmacokinetics after intranasal absorption. The bioavailability of Remimazolam by intranasal administration in animal no. 2 was 10% as assessed by cross-referencing to mean Remimazolam plasma profiles in female minipigs observed during and after intravenous infusion of 120 mg/kg over 6 hours in other studies (LPT study 32236 and associated bioanalytical report Aptuit VNG3585). A similar estimate evolves from a comparison with an intravenous bolus and infusion study in micropigs (study Y08AG004) (see table below).

**Table: Assessment of bioavailability using previous studies* as references**

| **Study** | **Dose (mg/kg)** | **AUC_{0→last} (ng*h/ml)** | **AUC_{0→last}/Dose (ng*h*kg/ml/mg)** | **Bioavailability¹ (0-tₗₐₛₜ) (%)** |
|---|---|---|---|---|
| Present (33659) | 1.25¹ | 19.92¹ | 15.94¹ | 10.5 (32236) |
| | | | | 5.43 (Y08AG004^{bolus}) |
| | | | | 5.63 (Y08AG004^{infusion}) |
| 32236 | 120 | 18215.23 | 151.79 | n.a |
| Y08AG004^{bolus} | 1 | 293.3² | 293.3² | n.a |
| Y08AG004^{infusion} | 2.4 | 676.7² | 282.9² | n.a |

| | | | | |
|---|---|---|---|---|
| * Study 32236 (14-Day DRF Study of CNS7056 in Göttingen Minpigs Following Repeated Intravenous 4-Hour Infusion) and Study Y08AG004 (Concentrations of ONO-2745 and ONO-IN-252 in plasma after single intravenous bolus administration or infusion of ONO-2745BS to micropigs) ¹ Values are calculated for minipig No. 2 (bw 20 kg) ² Data are for 0→∞. Comparison of AUC_{0→last} and AUC_{0→∞} is justified, as for Remimazolam, the modeled portion in 0→∞ and the portion not included in 0→last are small. | | | | |

### Conclusion

While one of three treated animals showed signs of transient sedation, there were no observations of adverse systemic reaction (clinical observations, mortality, food and water consumption, and body weights) following intranasal administration of remimazolam drug product at a per body weight dose of 1.25 mg/kg. Thus, it can be determined that the systemic NOAEL for intranasal administration of the dry drug product powder is ≥1.25 mg/kg in this study. The direct intensive contact of 25 mg of the dry remimazolam drug product powder with the nasal mucosa of one nostril was tolerated with no indication of any change in the appearance of mucosal surfaces including those regions that had been in direct contact with the dry drug powder. Therefore, it can be concluded that NOAEL for local tolerability is ≥ 25 mg/animal. The plasma levels of Remimazolam (CNS7056) and the metabolite CNS7054 were below the lower limit of quantification of the GLP-validated bioanalytical method (20 ng/mL CNS7056, 100 ng/mL CNS7054) in all samples except of the sample obtained from animal no. 2 at 0.25 hours p.a. in which Remimazolam was found at 50.4 ng/mL.

Overall, it can be concluded that minipigs can be sedated by intranasal administration of remimazolam lyophilisated powder, and in the applicability of the lyophilisate its high hygroscopicity is a factor to be considered.

### Figure Legends

- Fig. 1:: Clinical observations in rats upon administration of Remimazolam. Fig. 1a: Rolling gait. Fig. 1b: subdued behaviour. Fig. 1c: decreased activity. In each Figure x-axis indicates the time of observation (time intervals 1 to 5 correspond to IPD, 5 min, 10 min, 15 min and 30 min) and y-axis indicates the number of animals showing a particular clinical observation.
- Fig. 2:: Time to Tail Flick. Analgesic response to increasing concentrations of inhaled remifentanil and/or remimazolam for 5 min as measured by time to tail flick, Maximum test duration 20 sec. n=5/group unless otherwise noted. Shown as mean with interquartile range. *** indicates significant difference from pre-test baseline (p<0.0001) and inhaled saline p=0.0002. **** indicates significant difference from baseline and saline control (p<0.0001)

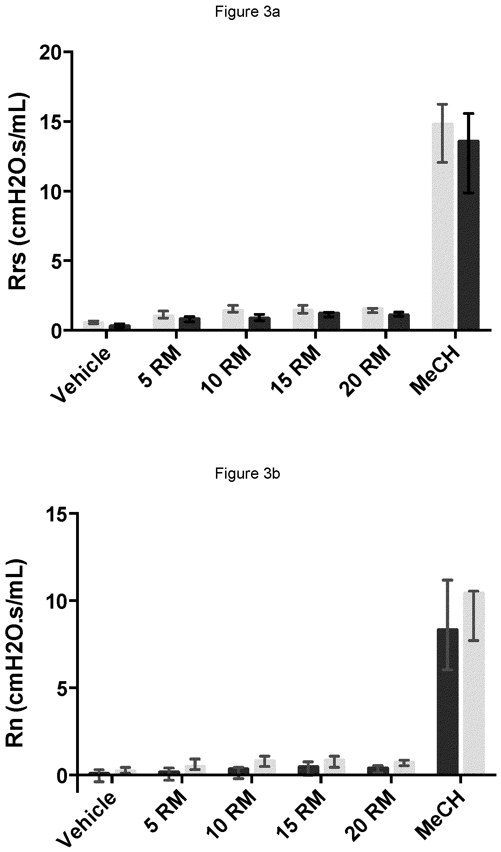

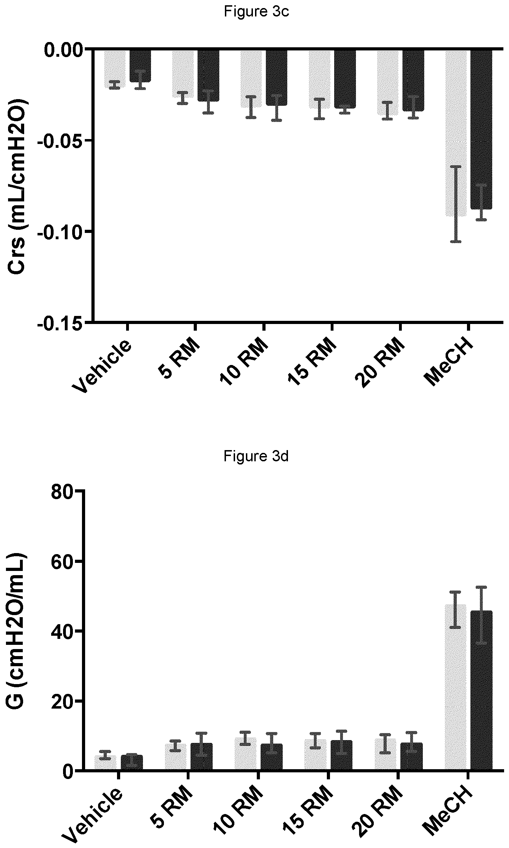

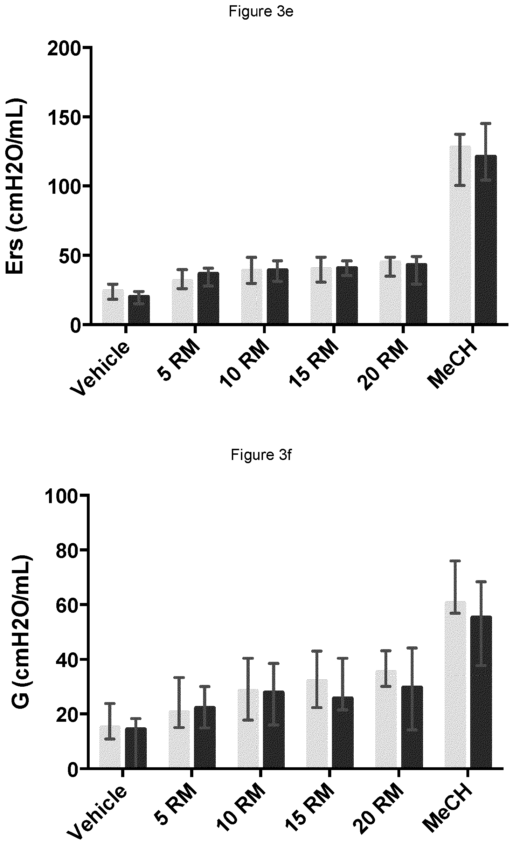

- Fig. 3:: Pulmonary mechanic measurements after exposure to increasing concentrations of inhaled remimazolam followed by methacholine challenge of C57BI/6 mice acutely exposed to increasing concentrations of inhaled remimazolam (mg/mL RM) as compared to inhaled vehicle (10 % DMSO/ 90 % saline) exposure, followed by 25 mg/mL methacholine (MeCH) challenge. n=5. Fig. 3a: Dose response of lung resistance (Rrs). Fig. 3b: Dose response of airway resistance (Rn). Fig. 3c: Dose response of lung compliance (Crs). Fig. 3d: Dose response of tissue damping or resistance (G). Fig. 3e: Dose response of lung elastance (Ers). Fig. 3f: Dose response of tissue elastance (H). In each Figure the x-axis indicates the treatment and the y-axis indicates measured pulmonary mechanics. The grey bars indicate mice administered vehicle 5-times followed by a methacholine challenge. The black bars indicate mice exposed to vehicle then to increasing concentrations of remimazolam followed by a methacholine challenge.

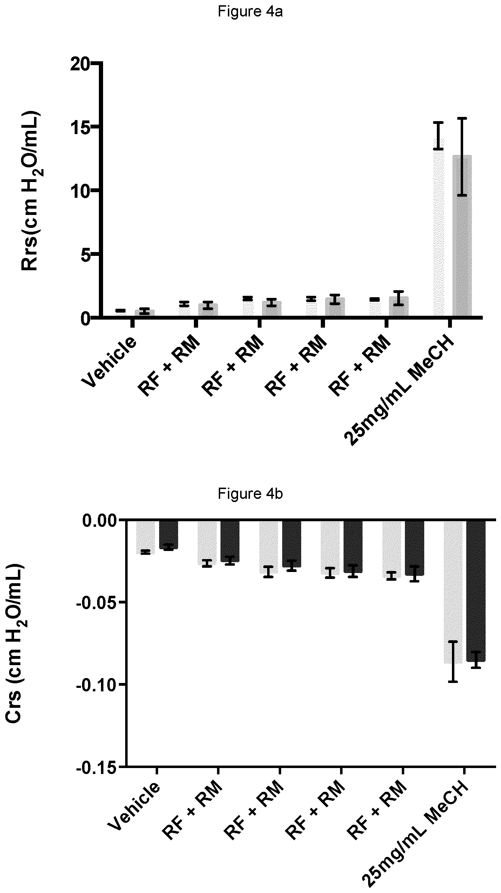

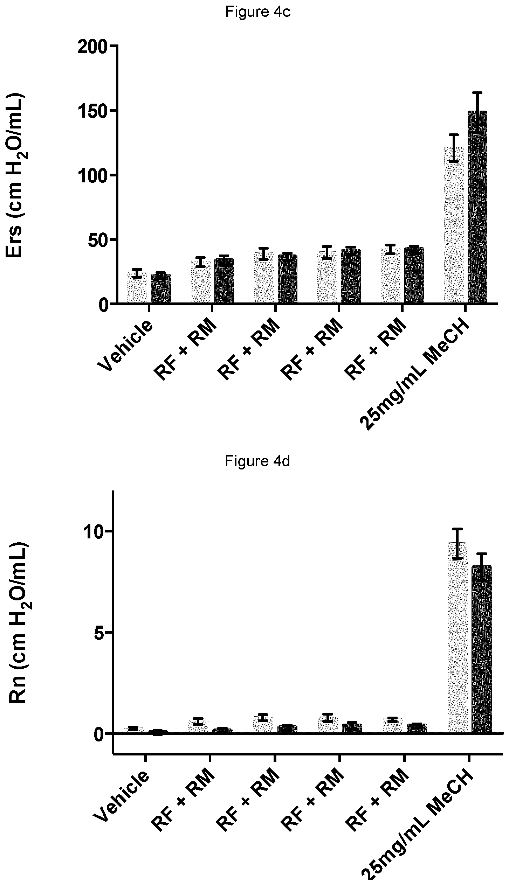

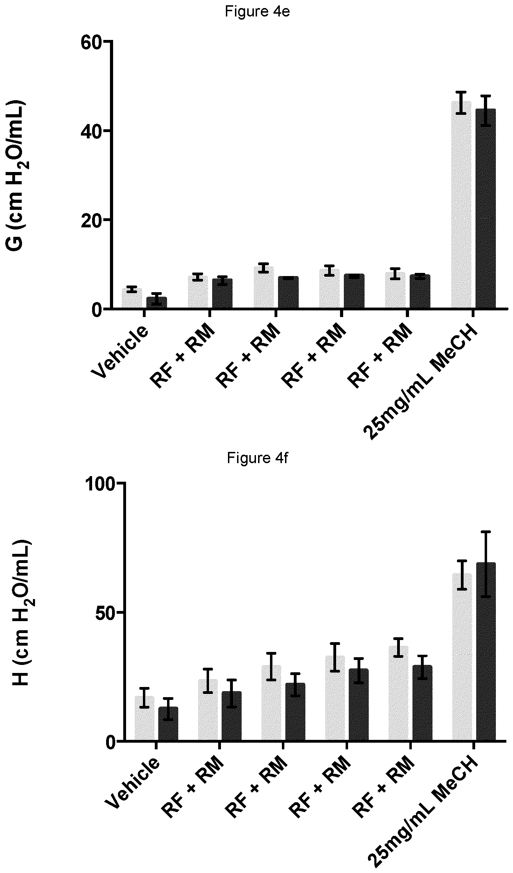

- Fig. 4:: Pulmonary mechanics measurements after acute exposure to a combination of inhaled remimazolam and remifentanil followed by methacholine challenge of C57BI/6 mice repeatedly exposed to 200 mcg/mL remifentanil (RF) combined with 20 mg/mL remimazolam (RM) by inhalation (grey bars) as compared to inhaled vehicle (10 % DMSO/ 90 % saline) exposure, followed by methacholine (MeCH) challenge (black bars). n=5. Fig. 4a: Lung resistance (Rrs). Fig. 4b: Lung compliance (Crs). Fig. 4c: Lung elastance (Ers). Fig. 4d: Airway resistance (Rn). Fig. 4e: Tissue damping or resistance (G). Fig. 4f: Tissue elastance (H). In each Figure the x-axis indicates the treatment and the y-axis indicates measured pulmonary mechanics.

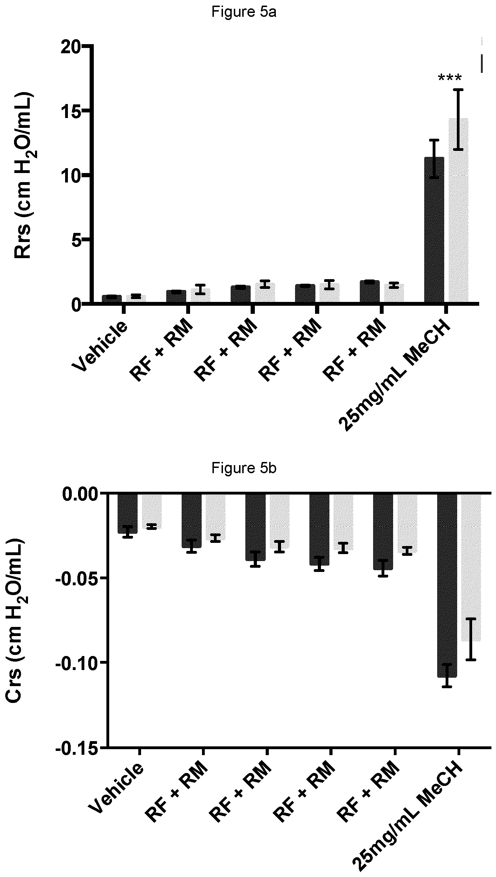

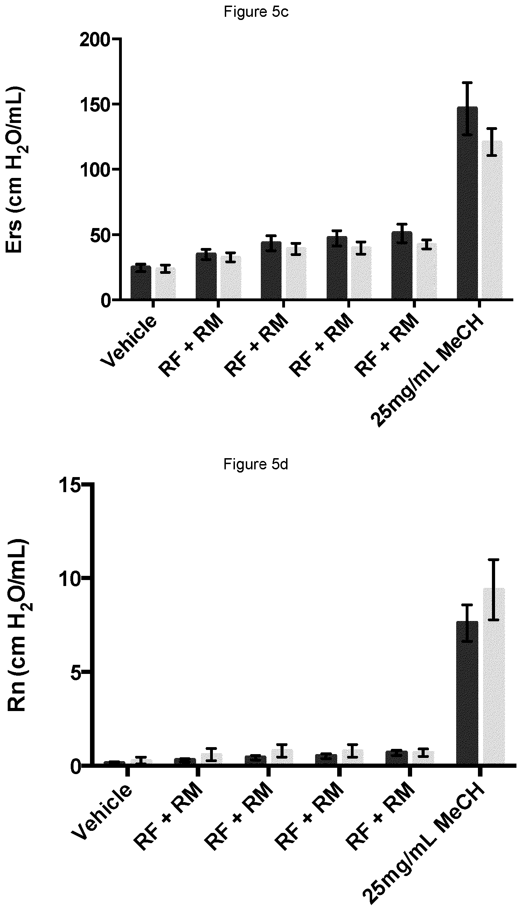

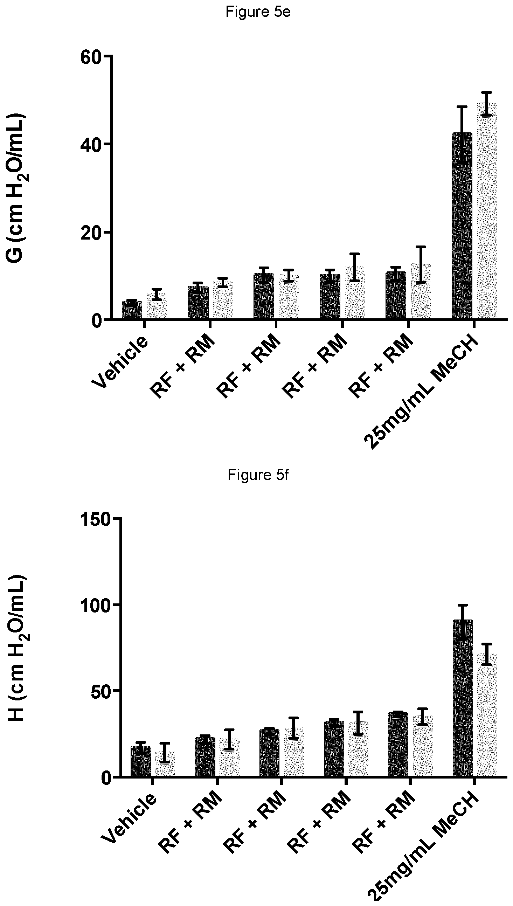

- Fig. 5:: Pulmonary mechanics measurements following exposure to sub-acute combination of inhaled remimazolam and remifentanil followed by methacholine challenge of C57BI/6 mice after repeated pulmonary exposure to 200 mcg/mL remifentanil (RF) and 20 mg/mL remimazolam (RM) with repeat prior exposure to inhaled RF and RM (grey bars) as compared to mice with repeated exposure to inhaled vehicle, followed by methacholine (MeCH) challenge (black bars). n=5, ***=P<0.0007. Fig. 5a: Lung resistance (Rrs). Fig. 5b: Lung compliance (Crs). Fig. 5c: Lung elastance (Ers). Fig. 5d: Airway resistance (Rn). Fi. 5e: Tissue damping or resistance (G). Fig. 5f: Tissue elastance (H). In each Figure the x-axis indicates treatments and the y-axis indicates measured pulmonary mechanics.

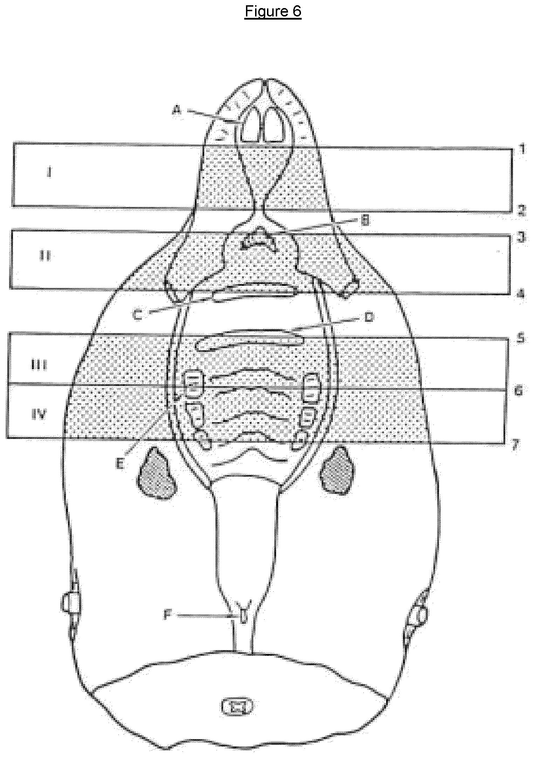

- Fig. 6:: Dissections I to IV taken from the nasal cavity of rats. Sections I - cross sectioned 2-3mm posterior to the upper incisor teeth. Section II - cross sectioned through the first palatal ridge. Section III - cross sectioned through the middle of the first upper molar teeth, passed through the anterior (medial) portion of both eye orbits. Section IV - cross sectioned through the third upper molar teeth.
- Fig. 7:: Sedative score in animal 2. Fig. 7a: Symptom 1 (acoustic or light stimulus). Fig. 7b: Symptom 2 (mechanical stimulus).

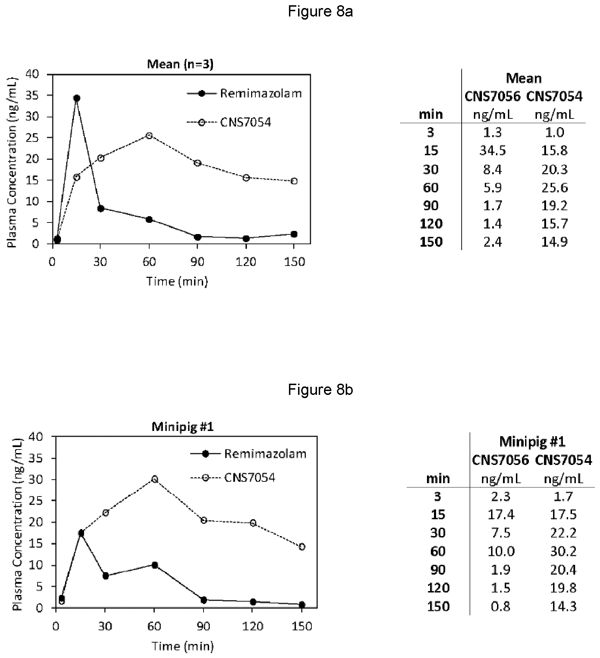

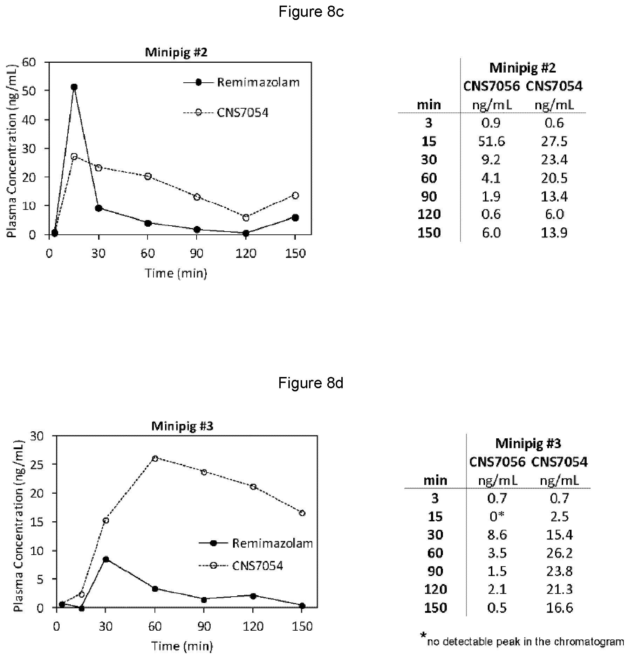

- Fig. 8:: Pharmacokinetic (linear extrapolation to zero, non-GLP) profiles of animal data. The plasma concentrations of remimazolam and CNS 7054 are given. Fig. 8a: Mean. Fig. 8b-d: Animals 1, 2 and 3, respectively.

The present invention also relates to the following embodiments:
1. A benzodiazepine according to formula (I) 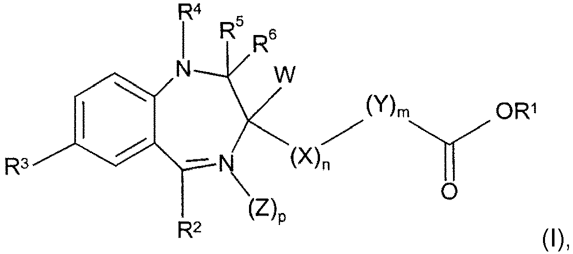 wherein
   W is H;
   X is CH₂; n is 1;
   Y is CH₂; m is 1;
   Z is O;
   R' is CH₃, CH₂CH₃, CH₂CH₂CH₃, CH(CH₃)₂ or CH₂CH(CH₃)₂;
   R² is 2-fluorophenyl, 2-chlorophenyl or 2-pyridyl;
   R³ is Cl or Br;
   R⁴, R⁵ and R⁶ form the group-CR⁸=U-V= wherein R⁸ is hydrogen, C₁₋₄ alkyl or C₁₋₃ hydroxyalkyl, U is N or CR⁹ wherein R⁹ is H, C₁₋₄ alkyl, C₁₋₃ hydroxyalkyl or C₁₋₄ alkoxy, V is N or CH and p is zero,
   or a pharmaceutically acceptable salt thereof,
   for use as a medicament, wherein the medicament is an orally inhaled and nasal drug product (OINDP).
2. The benzodiazepine or salt for the use according to embodiment 1, wherein the medicament is administered intranasally.
3. The benzodiazepine or salt for the use according to embodiment 1 or 2, wherein the medicament is administered as nasal drops, a nasal spray, a nasal aerosol, a nasal emulsion, an ointment, a gel, a paste, a cream, or as a powder.
4. The benzodiazepine or salt for the use according to embodiment 1, wherein the medicament is administered intrapulmonary.
5. The benzodiazepine or salt for the use according to embodiment 4, wherein the medicament is administered as an inhalation aerosol, an inhalation spray, a nebulized inhalation solution or suspension, or a dry powder.
6. The benzodiazepine or salt for the use according to any one of embodiments 1 to 5, wherein the medicament comprises a further active ingredient or is co-administered with a further active ingredient, wherein the further active ingredient is preferably an analgesic.
7. The benzodiazepine or salt for the use according to any one of embodiments 1 to 6, wherein the medicament comprises a formulation which comprises a vehicle, a carrier and/or one or more excipients.
8. The benzodiazepine or salt for the use according to embodiment 7, wherein the carrier or the one or more excipients comprise a polymer, preferably a polysaccharide or a polyether, more preferably chitosan or polyethylene glycol.
9. The benzodiazepine or salt for the use according to embodiment 8, wherein the amount of chitosan is 0.1 to 5 % by weight relative to the total amount of the medicament or wherein the amount of polyethylene glycol is 1 to 40 % by weight relative to the total amount of the medicament.
10. The benzodiazepine or salt for the use according to any one of embodiments 7 to 9, wherein the vehicle is aqueous or gaseous and/or wherein the carrier is particulate.
11. The benzodiazepine or salt for the use according to embodiment 10, wherein the carrier comprises microparticles or nanoparticles.
12. The benzodiazepine or salt for the use according to any one of embodiments 1 to 11, which is contained in an aqueous formulation having a pH value of 3 to 9.
13. The benzodiazepine or salt for the use according to any one of embodiments 1 to 12, which is contained in a liquid formulation having a viscosity of 2 to 50 mPa*s.
14. The benzodiazepine or salt for the use according to any one of embodiments 1 to 13, wherein the medicament is administered to obtain a systemic effect.
15. The benzodiazepine or salt for the use according to any one of embodiments 1 to 14, wherein the medicament is for use in sedation, hypnosis, anxiolysis, anesthesia, muscle relaxation or the treatment of convulsions.
16. The benzodiazepine or salt for the use according to any one of embodiments 1 to 15, wherein the medicament is for sedating or calming a patient selected from the group consisting of:
   (i) Children and infants undergoing a diagnostic or surgical procedure;
   (ii) children and infants prior to receiving intravenous anaesthesia or inhalational anaesthesia;
   (iii) patients for whom an intravenous access is difficult or impossible; and
   (iv) patients who suffer from a panic attack or epilepsy.
17. A device including a benzodiazepine according to formula (I) 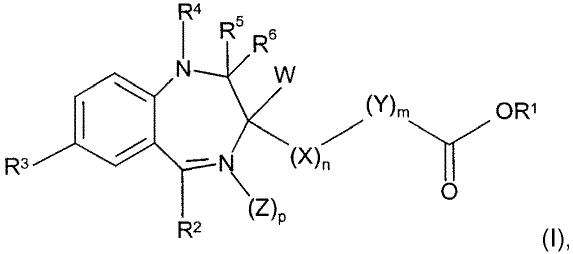 wherein
   W is H;
   X is CH₂; n is 1;
   Y is CH₂; m is 1;
   Z is O;
   R' is CH₃, CH₂CH₃, CH₂CH₂CH₃, CH(CH₃)₂ or CH₂CH(CH₃)₂;
   R² is 2-fluorophenyl, 2-chlorophenyl or 2-pyridyl;
   R³ is Cl or Br;
   R⁴, R⁵ and R⁶ form the group-CR⁸=U-V= wherein R⁸ is hydrogen, C₁₋₄ alkyl or C₁₋₃ hydroxyalkyl, U is N or CR⁹ wherein R⁹ is H, C₁₋₄ alkyl, C₁₋₃ hydroxyalkyl or C₁₋₄ alkoxy, V is N or CH and p is zero,
   or a pharmaceutically acceptable salt thereof, wherein the device is adapted for administering an orally inhaled or nasal drug product (OINDP) comprising the benzodiazepine or salt.
18. The device according to embodiment 17 which is a device for intranasal delivery selected from the group consisting of spray pump systems, pipettes for delivering drops, metered-dose spray pumps, nasal pressurized metered-dose inhalers, powder spray systems, breath-actuated powder inhalers and nasal powder insufflators or a device for intrapulmonary delivery selected from the group consisting of a metered dose inhaler, a dry powder inhaler and a nebulizer.
19. The device according to embodiments 17 or 18 which is dose-metered.
20. The device according to embodiment 19, wherein the dose-meter releases the benzodiazepine or pharmaceutically acceptable salt thereof, in individual doses of 5 to 250 mg.
21. A composition comprising a benzodiazepine according to formula (I) 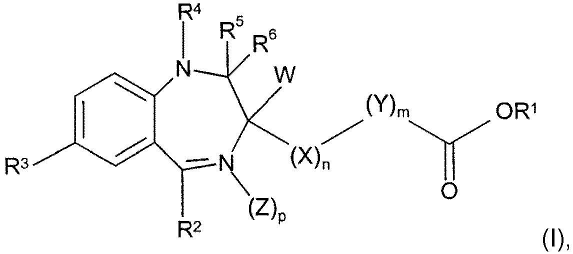 wherein
   W is H;
   X is CH₂; n is 1;
   Y is CH₂; m is 1;
   Z is O;
   R¹ is CH₃, CH₂CH₃, CH₂CH₂CH₃, CH(CH₃)₂ or CH₂CH(CH₃)₂;
   R² is 2-fluorophenyl, 2-chlorophenyl or 2-pyridyl;
   R³ is Cl or Br;
   R⁴, R⁵ and R⁶ form the group-CR⁸=U-V= wherein R⁸ is hydrogen, C₁₋₄ alkyl or C₁₋₃ hydroxyalkyl, U is N or CR⁹ wherein R⁹ is H, C₁₋₄ alkyl, C₁₋₃ hydroxyalkyl or C₁₋₄ alkoxy, V is N or CH and p is zero,
   or a pharmaceutically acceptable salt thereof,
   and at least one substance selected from the group consisting of a propellant, microparticles, nanoparticles, a mucoadhesive, a polyether, in particular polyethylene glycol (PEG), more particularly PEG 400, and a polysaccharide, in particular chitosan.
22. The composition according to embodiment 21 which is a pharmaceutical formulation, in particular an intranasal or an intrapulmonary formulation.
23. A benzodiazepine or salt for the use according to any one of embodiments 1 to 16, or a device according to any one of embodiments 17 to 20, or a composition according to embodiment 21 or 22, wherein the benzodiazepine is methyl 3-[(4S)-8-bromo-1-methyl-6-(pyridin-2-yl)-4H-imidazo[1,2-a][1,4]benzodiazepin-4-yl]propanoate (remimazolam), or a pharmaceutically acceptable salt thereof.
24. The benzodiazepine or salt for the use according to embodiment 23, or the device or composition according to embodiment 23, wherein the pharmaceutically acceptable salt of the benzodiazepine is present in cationic form and the counter ion is selected from the group consisting of halogenides, in particular fluoride, chloride or bromide, sulfate, organic sulfates, sulfonate, organic sulfonates, nitrate, phosphate, salicylate, tartrate, citrate, maleate, formiate, malonate, succinate, isethionate, lactobionate and sulfamate.
25. The benzodiazepine or salt for the use according to embodiment 24 or the device or composition according to embodiment 24, wherein the counter ion is selected from organic sulfates and sulfonates, in particular aromatic sulfates and sulfonates.
26. The benzodiazepine or salt for the use according to embodiment 25, or the device or composition according to embodiment 25, wherein the counter ion is benzene sulfonate (besylate).
27. The benzodiazepine or salt for the use according to embodiment 26, or the device or composition according to embodiment 26, wherein the pharmaceutically acceptable salt of the benzodiazepine is remimazolam besylate.
28. The benzodiazepine or salt for the use according to embodiment 25, or the device or composition according to embodiment 25, wherein the pharmaceutically acceptable salt of the benzodiazepine is a tosylate, in particular remimazolam tosylate.
29. The benzodiazepine or salt for the use according to embodiment 25, or the device or composition according to embodiment 25, wherein the pharmaceutically acceptable salt of the benzodiazepine is an esylate, in particular remimazolam esylate.
30. A benzodiazepine according to formula (I) wherein 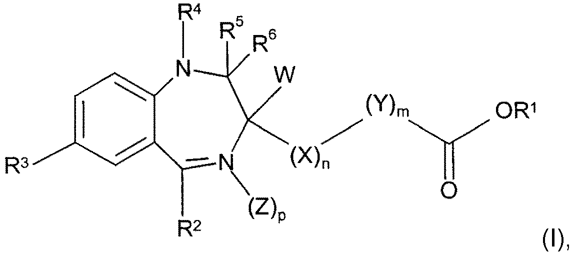
   W is H;
   X is CH₂; n is 1;
   Y is CH₂; m is 1;
   Z is O;
   R' is CH₃, CH₂CH₃, CH₂CH₂CH₃, CH(CH₃)₂ or CH₂CH(CH₃)₂;
   R² is 2-fluorophenyl, 2-chlorophenyl or 2-pyridyl;
   R³ is Cl or Br;
   R⁴, R⁵ and R⁶ form the group-CR⁸=U-V= wherein R⁸ is hydrogen, C₁₋₄ alkyl or C₁₋₃ hydroxyalkyl, U is N or CR⁹ wherein R⁹ is H, C₁₋₄ alkyl, C₁₋₃ hydroxyalkyl or C₁₋₄ alkoxy, V is N or CH and p is zero,
   or a pharmaceutically acceptable salt thereof,
   for use by intrapulmonary and/or intranasal administration of the benzodiazepine or salt.
31. The benzodiazepine or salt for use according to any of embodiments 1 to 16, wherein the medicament is administered in a single dose or in multiple doses.
32. The benzodiazepine or salt for use according to embodiment 31, which is administered in a single dose or in multiple doses.

## Claims

1. A benzodiazepine according to formula (I) 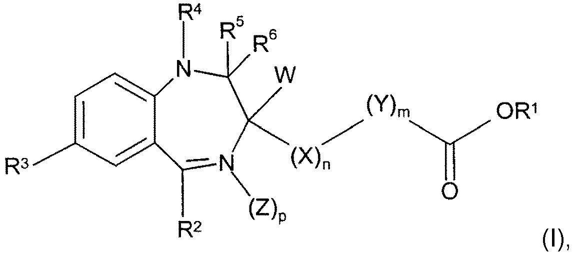 wherein
W is H;
X is CH₂; n is 1;
Y is CH₂; m is 1;
Z is O;
R¹ is CH₃, CH₂CH₃, CH₂CH₂CH₃, CH(CH₃)₂ or CH₂CH(CH₃)₂;
R² is 2-fluorophenyl, 2-chlorophenyl or 2-pyridyl;
R³ is Cl or Br;
R⁴, R⁵ and R⁶ form the group-CR⁸=U-V= wherein R⁸ is hydrogen, C₁₋₄ alkyl or C₁₋₃ hydroxyalkyl, U is N or CR⁹ wherein R⁹ is H, C₁₋₄ alkyl, C₁₋₃ hydroxyalkyl or C₁₋₄ alkoxy, V is N or CH and p is zero,
or a pharmaceutically acceptable salt thereof,
for use as a medicament by administration to the respiratory tract, in particular to the lung and/or nasal structures, wherein the medicament is
a) for use in sedation, hypnosis, anxiolysis, anesthesia, muscle relaxation or the treatment of convulsions, or
b) for sedating or calming a patient selected from the group consisting of:
(i) Children and infants undergoing a diagnostic or surgical procedure;
(ii) children and infants prior to receiving intravenous anaesthesia or inhalational anaesthesia;
(iii) patients for whom an intravenous access is difficult or impossible; and
(iv) patients who suffer from a panic attack or epilepsy.

2. A benzodiazepine according to formula (I) 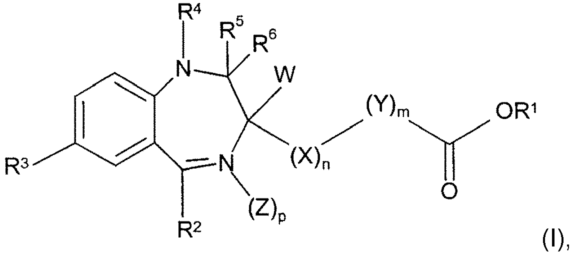 wherein
W is H;
X is CH₂; n is 1;
Y is CH₂; m is 1;
Z is O;
R¹ is CH₃, CH₂CH₃, CH₂CH₂CH₃, CH(CH₃)₂ or CH₂CH(CH₃)₂;
R² is 2-fluorophenyl, 2-chlorophenyl or 2-pyridyl;
R³ is Cl or Br;
R⁴, R⁵ and R⁶ form the group-CR⁸=U-V= wherein R⁸ is hydrogen, C₁₋₄ alkyl or C₁₋₃ hydroxyalkyl, U is N or CR⁹ wherein R⁹ is H, C₁₋₄ alkyl, C₁₋₃ hydroxyalkyl or C₁₋₄ alkoxy, V is N or CH and p is zero,
or a pharmaceutically acceptable salt thereof,
for use as a medicament, wherein administration of the medicament leads to oral absorption followed by hepatic first pass metabolism, wherein the medicament is
a) for use in sedation, hypnosis, anxiolysis, anesthesia, muscle relaxation or the treatment of convulsions, or
b) for sedating or calming a patient selected from the group consisting of:
(i) Children and infants undergoing a diagnostic or surgical procedure;
(ii) children and infants prior to receiving intravenous anaesthesia or inhalational anaesthesia;
(iii) patients for whom an intravenous access is difficult or impossible; and
(iv) patients who suffer from a panic attack or epilepsy.

3. The benzodiazepine or salt for the use according to claim 1 or 2, wherein the medicament comprises a formulation which comprises a vehicle, a carrier and/or one or more excipients.

4. The benzodiazepine or salt for the use according to claim 3, wherein the carrier or the one or more excipients comprise a polymer, preferably a polysaccharide or a polyether, more preferably chitosan or polyethylene glycol.

5. The benzodiazepine or salt for the use according to claim 4, wherein the amount of chitosan is 0.1 to 5 % by weight relative to the total amount of the medicament or wherein the amount of polyethylene glycol is 1 to 40 % by weight relative to the total amount of the medicament.

6. The benzodiazepine or salt for the use according to any one of claims 1 to 5, which is contained in an aqueous formulation having a pH value of 3 to 9.

7. The benzodiazepine or salt for the use according to any one of claims 1 to 6, which is contained in a liquid formulation having a viscosity of 2 to 50 mPa*s.

8. The benzodiazepine or salt for the use according to any one of claims 1 to 7, wherein the medicament is administered to obtain a systemic effect.

9. A composition comprising a benzodiazepine according to formula (I) 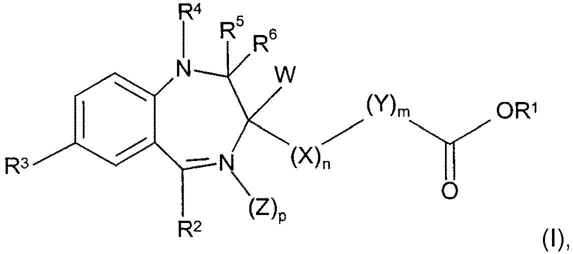 wherein
W is H;
X is CH₂; n is 1;
Y is CH₂; m is 1;
Z is O;
R' is CH₃, CH₂CH₃, CH₂CH₂CH₃, CH(CH₃)₂ or CH₂CH(CH₃)₂;
R² is 2-fluorophenyl, 2-chlorophenyl or 2-pyridyl;
R³ is Cl or Br;
R⁴, R⁵ and R⁶ form the group-CR⁸=U-V= wherein R⁸ is hydrogen, C₁₋₄ alkyl or C₁₋₃ hydroxyalkyl, U is N or CR⁹ wherein R⁹ is H, C₁₋₄ alkyl, C₁₋₃ hydroxyalkyl or C₁₋₄ alkoxy, V is N or CH and p is zero,
or a pharmaceutically acceptable salt thereof,
and at least one substance selected from the group consisting of a propellant, microparticles, nanoparticles, a mucoadhesive, a polyether, in particular polyethylene glycol (PEG), more particularly PEG 400, a polysaccharide, in particular chitosan, and a viscosifying agent, in particular selected from the group consisting of hydroxyethylcellulose, hydroxypropylmethyl-cellulose, methylcellulose, carboxymethylcellulose, ethylcellulose, polyvinyl alcohol, polyvinylpyrrolidone, carboxy-vinyl polymer, carrageenan and carbopol.

10. The composition according to claim 9 which is a pharmaceutical formulation.

11. A benzodiazepine or salt for the use according to any one of claims 1 to 8, or a composition according to claim 9 or 10, wherein the benzodiazepine is methyl 3-[(4S)-8-bromo-1-methyl-6-(pyridin-2-yl)-4H-imidazo[1,2-a][1,4]benzodiazepin-4-yl]propanoate (remimazolam), or a pharmaceutically acceptable salt thereof.

12. The benzodiazepine or salt for the use according to claim 11, or the composition according to claim 11, wherein the pharmaceutically acceptable salt of the benzodiazepine is present in cationic form and the counter ion is selected from the group consisting of halogenides, in particular fluoride, chloride or bromide, sulfate, organic sulfates, sulfonate, organic sulfonates, nitrate, phosphate, salicylate, tartrate, citrate, maleate, formiate, malonate, succinate, isethionate, lactobionate and sulfamate.

13. The benzodiazepine or salt for the use according to claim 12 or the composition according to claim 12, wherein the counter ion is selected from organic sulfates and sulfonates, in particular aromatic sulfates and sulfonates.

14. The benzodiazepine or salt for the use according to claim 13, or the composition according to claim 13, wherein the counter ion is benzene sulfonate (besylate) and the pharmaceutically acceptable salt of the benzodiazepine is preferably remimazolam besylate.

15. The benzodiazepine or salt for the use according to claim 25, or the composition according to claim 25, wherein the pharmaceutically acceptable salt of the benzodiazepine is a tosylate, in particular remimazolam tosylate or an esylate, in particular remimazolam esylate.
